# EUROPEAN PATENT APPLICATION

(11) **EP 4 728 977 A1**
(43) Date of publication of application: **22.04.2026**
(21) Application number: 24884820.2
(22) Date of filing: 30.10.2024
(51) Int. Cl.: A61B 5/02, A61B 5/022, A61B 5/0225

(54) **BLOOD PRESSURE MEASUREMENT METHOD, WEARABLE DEVICE, AND STORAGE MEDIUM**

(30) Priority: 31.10.2023 CN 202311444031
(71) Applicant: Huawei Technologies Co., Ltd., Shenzhen, Guangdong 518129 (CN)
(72) Inventor: JIN, Junye, Shenzhen, Guangdong 518129 (CN); HUANG, Yadong, Shenzhen, Guangdong 518129 (CN); RONG, Meng, Shenzhen, Guangdong 518129 (CN); LU, Shiqiang, Shenzhen, Guangdong 518129 (CN)
(74) Representative: Isarpatent
(86) International application number: PCT/CN2024/128504
(87) International publication number: WO 2025/092820

(57) **Abstract**

A blood pressure measurement method applied to a wearable device, a wearable device to which the blood pressure measurement method is applied, and a storage medium are disclosed. The wearable device (100) includes a blood pressure measurement apparatus (213). The blood pressure measurement apparatus (213) includes an airbag (213G). Within a first time period, the wearable device (100) inflates the airbag (213G), and obtains a first pulse wave signal, where pressure in the airbag (213G) gradually increases over time within the first time period, and the first pulse wave signal is used to obtain a first blood pressure measurement value. Within a second time period, the wearable device inflates the airbag (213G), and obtains a second pulse wave signal, where within the second time period, pressure in the airbag (213G) remains unchanged, or pressure in the airbag (213G) fluctuates within a first range, and the second pulse wave signal is used to assess a cardiovascular risk. The wearable device (100) outputs the first blood pressure measurement value and the cardiovascular risk. According to the method, the wearable device (100) can further monitor a cardiovascular disease risk of a user when obtaining a peripheral arterial blood pressure value, to prevent occurrence of a cardiovascular disease.

## Description

This application claims priority to Chinese Patent Application No. 202311444031.1, filed with the China National Intellectual Property Administration on October 31, 2023 and entitled "BLOOD PRESSURE MEASUREMENT METHOD, WEARABLE DEVICE, AND STORAGE MEDIUM", which is incorporated herein by reference in its entirety.

### TECHNICAL FIELD

This application relates to the field of wearable technologies, and in particular, to a blood pressure measurement method, a wearable device, and a storage medium.

### BACKGROUND

With improvement of living standards, people's health attracts increasing attention, and cardiovascular diseases of users also become increasingly common. The cardiovascular diseases are one of primary factors that cause an increase in a death risk. It is found through research that hypertension is also one of hazardous factors that cause cardiovascular diseases of residents. Monitoring blood pressure and predicting a cardiovascular risk are of great significance.

To conveniently monitor blood pressure, a wrist dynamic blood pressure monitor is available. The wrist dynamic blood pressure monitor is comfortable to wear, and helps save time and effort. The wrist dynamic blood pressure monitor may monitor blood pressure of a user within 24 hours. The wrist dynamic blood pressure monitor may measure blood pressure by using an oscillometric method. For example, the wrist dynamic blood pressure monitor may perform inflation with linearly increasing pressure, to block arterial blood flow by using air and obtain oscillation waves of different pressure, to determine information such as systolic pressure and diastolic pressure. However, the wrist dynamic blood pressure monitor cannot predict one or more of the following cardiovascular risks: a heart rate, vascular wall elasticity, a cardiac ejection capability, peripheral vascular resistance, and the like. How to enable the wrist dynamic blood pressure monitor to predict a cardiovascular risk while monitoring blood pressure is to be further studied.

### SUMMARY

This application provides a blood pressure measurement method, a wearable device, and a storage medium, to enable a wearable device to further obtain a central arterial pulse wave signal when obtaining a peripheral arterial blood pressure value, and monitor a cardiovascular disease risk of a user based on the central arterial pulse wave signal, to prevent occurrence of a cardiovascular disease.

According to a first aspect, this application provides a blood pressure measurement method. The method is applied to a wearable device. The wearable device includes a blood pressure measurement apparatus. The blood pressure measurement apparatus includes an airbag. The method includes: Within a first time period, the wearable device inflates the airbag and obtains a first pulse wave signal, where pressure in the airbag gradually increases over time within the first time period, and the first pulse wave signal is used to obtain a first blood pressure measurement value. Within a second time period, the wearable device inflates the airbag and obtains a second pulse wave signal, where within the second time period, pressure in the airbag remains unchanged, or pressure in the airbag fluctuates within a first range, and the second pulse wave signal is used to assess a cardiovascular risk. The wearable device outputs the first blood pressure measurement value and the cardiovascular risk.

The first blood pressure measurement value may be a blood pressure value, measured by the wearable device, of a part wearing the wearable device. For example, when the wearable device is worn on a wrist of a user, the first blood pressure measurement value may be a blood pressure value of the wrist of the user.

That the wearable device outputs the first blood pressure measurement value and the cardiovascular risk may include: The wearable device displays the first blood pressure measurement value and the cardiovascular risk, or plays the first blood pressure measurement value and the cardiovascular risk through speech, or sends the first blood pressure measurement value and the cardiovascular risk to another device for display.

According to the method, the wearable device can further monitor a cardiovascular disease risk of the user when obtaining a peripheral arterial blood pressure value, to prevent occurrence of a cardiovascular disease.

With reference to the first aspect, in a possible implementation, the method further includes: The wearable device obtains a third pulse wave signal based on the second pulse wave signal and a first target model. The wearable device obtains a second blood pressure measurement value based on the first blood pressure measurement value and the third pulse wave signal.

The first blood pressure measurement value may be a central arterial blood pressure value. The central arterial blood pressure value may be aortic blood pressure, and is pressure directly applied to the heart and other organs. The aorta is closer to the heart and the brain. Therefore, compared with the peripheral arterial blood pressure value, the central arterial blood pressure value is of greater significance in assessing a blood pressure risk of the user.

According to the method, the wearable device can further obtain the central arterial blood pressure value when obtaining the peripheral arterial blood pressure value.

With reference to the first aspect, in a possible implementation, the cardiovascular risk includes any one or more of the following: a heart rate, vascular wall elasticity, a cardiac ejection capability, and peripheral vascular resistance.

With reference to the first aspect, in a possible implementation, before the wearable device obtains the first blood pressure measurement value based on the first pulse wave signal, the method further includes: Within a third time period, the wearable device inflates the airbag and obtains a fourth pulse wave signal, where pressure in the airbag gradually increases over time within the third time period. The wearable device splices the first pulse wave signal and the fourth pulse wave signal in chronological order to obtain a fifth pulse wave signal, where the fifth pulse wave signal is used to obtain the first blood pressure measurement value.

Optionally, the wearable device may obtain the first blood pressure measurement value based on the fifth pulse wave signal and airbag pressure signals that correspond to the first time period and the third time period.

Measuring blood pressure by the wearable device is a complete process of inflation and deflation. In the complete process of inflation and deflation, the wearable device may obtain pulse wave signals in a plurality of linear pressure increase periods. The wearable device may splice the pulse wave signals in the plurality of linear pressure increase periods into a complete pulse wave signal, and obtain the first blood pressure measurement value based on the complete pulse wave signal.

With reference to the first aspect, in a possible implementation, before the wearable device outputs the first blood pressure measurement value and the cardiovascular risk, the method further includes: Within a fourth time period, the wearable device inflates the airbag and obtains a sixth pulse wave signal, where within the fourth time period, pressure in the airbag remains unchanged, or pressure in the airbag fluctuates within the first range. That the wearable device obtains the second pulse wave signal specifically includes: The wearable device selects the second pulse wave signal with a good signal from the second pulse wave signal and the sixth pulse wave signal.

Optionally, the wearable device may obtain the third pulse wave signal based on the second pulse wave signal and the first target model, and the wearable device obtains, based on the third pulse wave signal, parameter information for assessing the cardiovascular risk.

Measuring blood pressure by the wearable device is a complete process of inflation and deflation. In the complete process of inflation and deflation, the wearable device may obtain pulse wave signals in a plurality of constant pressure control periods.

In a possible implementation, the wearable device may select a pulse wave signal with a good signal from the pulse wave signals in the plurality of constant pressure control periods, obtain a central pulse wave signal based on the pulse wave signal, and then assess the cardiovascular risk based on the central pulse wave signal.

In another possible implementation, the wearable device may obtain a central pulse wave signal based on a pulse wave signal in each constant pressure control period, then assess the cardiovascular risk based on each central pulse wave signal, and finally average a plurality of cardiovascular risks to obtain a final cardiovascular risk.

With reference to the first aspect, in a possible implementation, the blood pressure measurement apparatus further includes a drive circuit, a micropump, and a pressure sensor. The drive circuit is configured to control an air inflation rate at which the micropump inflates the airbag. The pressure sensor is configured to obtain an arterial pulse wave signal. An air release rate of the micropump is a first air release rate. That the wearable device inflates the airbag and obtains the first pulse wave signal specifically includes: The wearable device controls the drive circuit to be in a first duty cycle, controls, through the drive circuit, the micropump to inflate the airbag at a first air inflation rate, and obtains the first pulse wave signal through the pressure sensor, where the first air inflation rate is greater than the first air release rate.

With reference to the first aspect, in a possible implementation, that the wearable device inflates the airbag and obtains the second pulse wave signal specifically includes: The wearable device controls the drive circuit to be in a second duty cycle, controls, through the drive circuit, the micropump to inflate the airbag at a second air inflation rate, and obtains the second pulse wave signal through the pressure sensor, where the second duty cycle is less than the first duty cycle, the second air inflation rate is less than the first air inflation rate, and the second air inflation rate is equal to the first air release rate.

With reference to the first aspect, in a possible implementation, the blood pressure measurement apparatus further includes an air valve, and the air valve is configured to exhaust air in the airbag. In a process of obtaining, by the wearable device, the first pulse wave signal and the second pulse wave signal, the method further includes: The wearable device closes the air valve.

In some embodiments, the wearable device may control, by adjusting an inflation speed of the micropump, pressure in the airbag to increase or remain constant, to obtain the first blood pressure measurement value and the cardiovascular risk.

With reference to the first aspect, in a possible implementation, the blood pressure measurement apparatus further includes a drive circuit, a micropump, a pressure sensor, and an air valve. The drive circuit is configured to control an air inflation rate at which the micropump inflates the airbag. The pressure sensor is configured to obtain an arterial pulse wave signal. The air valve is configured to exhaust air in the airbag. An air release rate of the micropump is a first air release rate. That the wearable device inflates the airbag and obtains the first pulse wave signal specifically includes:

The wearable device controls the drive circuit to be in a first duty cycle, controls, through the drive circuit, the micropump to inflate the airbag at a first air inflation rate, closes the air valve, and obtains the first pulse wave signal through the pressure sensor, where the first air inflation rate is greater than the first air release rate.

With reference to the first aspect, in a possible implementation, that the wearable device inflates the airbag and obtains the second pulse wave signal specifically includes: The wearable device controls the drive circuit to be in the first duty cycle, controls, through the drive circuit, the micropump to inflate the airbag at the first air inflation rate, opens the air valve, and exhausts air in the airbag at a second air release rate through the air valve, where the first air inflation rate is equal to a sum of the first air release rate and the second air release rate.

In some embodiments, the wearable device may adjust an inflation speed of the micropump to remain constant, and control an air release speed of the air valve to enable pressure in the airbag to increase or remain constant, to obtain the first blood pressure measurement value and the cardiovascular risk.

With reference to the first aspect, in a possible implementation, after the wearable device obtains the first pulse wave signal and the second pulse wave signal, the method further includes: The wearable device opens the air valve and exhausts air in the airbag through the air valve.

In this way, after blood pressure measurement is completed, the wearable device may open the air valve to accelerate exhaustion of the air in the airbag.

According to a second aspect, this application provides a wearable device. The wearable device includes a blood pressure measurement apparatus, a memory, and a processor. The blood pressure measurement apparatus and the memory are coupled to the processor. The memory is configured to store a computer program. When the processor invokes the computer program, the wearable device is enabled to perform the blood pressure detection method according to any possible implementation of any one of the foregoing aspects.

According to a third aspect, this application provides a computer-readable storage medium, including instructions. When the instructions are run on a wearable device, the wearable device is enabled to perform the blood pressure detection method according to any possible implementation of any one of the foregoing aspects.

According to a fourth aspect, this application provides a chip system. The chip system includes one or more processors. The processor is configured to invoke computer instructions, to perform the blood pressure detection method according to any possible implementation of any one of the foregoing aspects.

According to a fifth aspect, this application provides a computer program product including instructions. When the computer program product is run on a wearable device, the wearable device is enabled to perform the blood pressure detection method according to any possible implementation of any one of the foregoing aspects.

For descriptions of beneficial effects of the second aspect to the fifth aspect, refer to the descriptions of the beneficial effects of the first aspect. Details are not described herein again in this application.

### BRIEF DESCRIPTION OF DRAWINGS

FIG. 1 is a diagram of a user wearing a wearable device 100;
FIG. 2 is a diagram of a composition structure of a wearable device 100;
FIG. 3 is a diagram of a hardware structure of a wearable device 100;
FIG. 4 is a diagram of a composition structure of a blood pressure measurement apparatus 213 according to this application;
FIG. 5 is a diagram of a composition structure of an airbag, an air pump, and an air path communication component according to an embodiment of this application;
FIG. 6 is a diagram of five linear pressure increase periods and four constant pressure control periods obtained that are obtained by a wearable device 100;
FIG. 7 is a diagram of duty cycles corresponding to a drive circuit 213B within linear pressure increase periods and constant pressure control periods;
FIG. 8 is a diagram of average pressure increase rates of an airbag 213G within linear pressure increase periods and constant pressure control periods;
FIG. 9A and FIG. 9B are a schematic flowchart of a method for obtaining N linear pressure increase periods and N constant pressure control periods by a wearable device 100;
FIG. 10 is a diagram of duty cycles corresponding to a drive circuit 213B within linear pressure increase periods and constant pressure control periods;
FIG. 11 shows average pressure increase rates of an airbag 213G within linear pressure increase periods and constant pressure control periods;
FIG. 12A and FIG. 12B are a schematic flowchart of another method for obtaining N linear pressure increase periods and N constant pressure control periods by a wearable device 100;
FIG. 13 and FIG. 14 are diagrams of obtaining a complete peripheral arterial pulse wave signal;
FIG. 15 is a diagram of obtaining a peripheral arterial blood pressure value according to an embodiment of this application;
FIG. 16 and FIG. 17 are diagrams of obtaining a central arterial pulse wave signal;
FIG. 18 is a diagram of a central arterial pulse wave signal obtained within one cycle;
FIG. 19 is a diagram of collecting a peripheral arterial pulse wave signal through a PPG module;
FIG. 20A and FIG. 20B are a group of diagrams;
FIG. 21A to FIG. 21C are diagrams of displaying, by a wearable device 100, a blood pressure measurement value and parameter information for assessing a cardiovascular disease; and
FIG. 22 is a schematic flowchart of a blood pressure measurement method according to this application.

### DESCRIPTION OF EMBODIMENTS

The following clearly describes the technical solutions in embodiments of this application in detail with reference to the accompanying drawings. In the descriptions of embodiments of this application, "/" indicates or, unless otherwise specified. For example, A/B may indicate A or B. In this specification, "and/or" describes only an association relationship between associated objects, and indicates that three relationships may exist. For example, A and/or B may indicate the following three cases: Only A exists, both A and B exist, and only B exists. In addition, in the descriptions of embodiments of this application, "a plurality of" means two or more than two.

The terms "first" and "second" used below are merely intended for description, and shall not be understood as an indication or implication of relative importance or an implicit indication of a quantity of indicated technical features. Therefore, a feature limited by "first" or "second" may explicitly or implicitly include one or more features. In the descriptions of embodiments of this application, "a plurality of" means two or more than two, unless otherwise specified.

The term "user interface (user interface, UI)" in the following embodiments of this application is a medium interface for interaction and information exchange between an application or an operating system and a user. The user interface implements conversion between an internal form of information and a form acceptable to the user. The user interface is usually represented in a form of a graphical user interface (graphical user interface, GUI), and is a user interface that is related to a computer operation and that is displayed in a graphic manner. The user interface may be a visual interface element, for example, text, an icon, a button, a menu, a tab, a text box, a dialog box, a status bar, a navigation bar, or a widget, that is displayed on a display of an electronic device.

With development of electronic technologies, functions of wearable devices are continuously enhanced. For example, a wearable device like a wristband or a watch may provide a blood pressure measurement function, to help a user measure blood pressure of the user anytime and anywhere, to learn of a physical condition of the user.

In this application, blood pressure may be measured by using an oscillometric method with inflation. When obtaining a peripheral arterial blood pressure value, the wearable device 100 may further add one or more constant pressure control periods, and pressure in an airbag remains unchanged within the constant pressure control period. The wearable device 100 may obtain a peripheral arterial pulse wave signal within the one or more constant pressure control period, and obtain a central arterial pulse wave signal based on the peripheral arterial pulse wave signal within the one or more constant pressure control period. The wearable device 100 may perform feature analysis on the central arterial pulse wave signal to obtain parameter information for assessing a cardiovascular risk. The parameter information for assessing the cardiovascular risk includes but is not limited to a heart rate, vascular wall elasticity, a cardiac ejection capability, peripheral vascular resistance, and the like.

In some embodiments, the peripheral arterial pulse wave signal may also be referred to as a peripheral pulse wave signal, and the central arterial pulse wave signal may also be referred to as a central pulse wave signal.

It should be noted that, within the constant pressure control period, the pressure in the airbag may remain unchanged, or may fluctuate within a specific range. For example, when pressure at a constant pressure control point is 90 mmHg millimeters of mercury, the pressure in the airbag within the constant pressure control period may fluctuate within a range of 85 mmHg to 95 mmHg.

In some embodiments, the wearable device may further obtain a central arterial blood pressure value based on the peripheral arterial blood pressure value and the central arterial pulse wave signal, and the central arterial blood pressure value can better indicate an actual blood pressure value of the user.

The oscillometric method with inflation may be: obtaining, through inflation with linearly increasing pressure, a continuous change status of arterial pressure along with inflation pressure, measuring blood pressure by blocking arterial blood flow, and calculating a peripheral arterial blood pressure value and other data.

The peripheral arterial blood pressure value may include peripheral arterial systolic pressure, peripheral arterial diastolic pressure, peripheral arterial average pressure, and the like. The peripheral arterial blood pressure value may be a blood pressure value, detected by the wearable device 100, of a part wearing the wearable device 100. For example, if the wearable device 100 is worn on a wrist of the user, the peripheral arterial blood pressure value may be a blood pressure value, detected by the wearable device 100, of the wrist.

The central arterial blood pressure value may include central arterial systolic pressure, central arterial diastolic pressure, central arterial average pressure, and the like. The central arterial blood pressure value may be aortic blood pressure, and is pressure directly applied to the heart and other organs. The aorta is closer to the heart and the brain. Therefore, compared with the peripheral arterial blood pressure value, the central arterial blood pressure value is of greater significance in assessing a blood pressure risk of the user.

According to the method, the wearable device 100 may obtain the central arterial blood pressure value of the user through measurement, to improve accuracy of blood pressure measurement. In addition, the wearable device 100 may obtain the central arterial pulse wave signal, and monitor a cardiovascular disease risk of the user based on the central arterial pulse wave signal, to prevent occurrence of a cardiovascular disease.

The following describes a wearable device 100, provided in this application, that can be used to measure blood pressure.

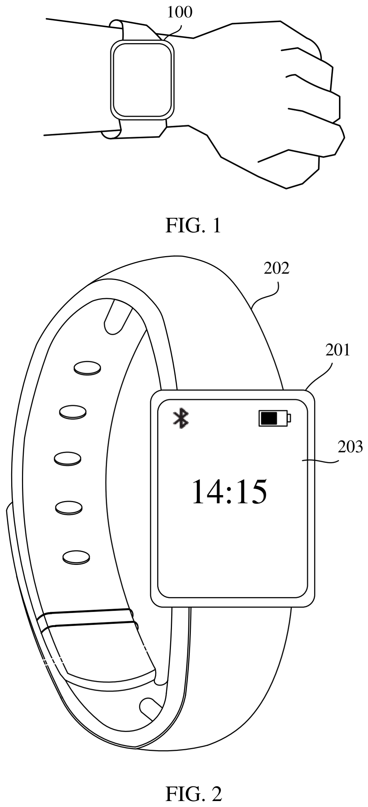

FIG. 1 is a diagram of a user wearing the wearable device 100.

As shown in FIG. 1, the user may wear the wearable device 100 on a wrist of the user.

FIG. 2 is a diagram of a composition structure of the wearable device 100.

As shown in FIG. 2, the wearable device 100 may include a watch body 201 and a wearable component 202.

The watch body 201 is equipped with a motion sensor, for example, a gyroscope sensor and an acceleration sensor. The motion sensor is configured to collect motion data, and determine a status of the user based on a motion status obtained through analysis based on the motion data.

The watch body 201 may include a display 203. The display 203 may be configured to display time, a battery level of the watch body 201, a Bluetooth identifier, a received message, motion data of the user, and other content. The display 203 may be configured to receive a tap operation of the user to turn on the display, enable or disable a sport mode, or the like. The display 203 may further record a quantity of moving steps and consumed calories of the user, and has basic functions such as an incoming call reminder and a message notification. In a possible implementation, the watch body 201 may establish a wireless communication connection to the wearable device 100 through Bluetooth. The watch body 201 may send the motion data of the user to the wearable device 100 to which the connection is established. In addition, when the wearable device 100 receives an incoming call or a message notification, the watch body 201 may receive an instruction from a mobile phone, to inform the user of the incoming call or the message notification.

The wearable component 202 is used for mounting the watch body 201. For example, the wearable component 202 may be a wristband strap, a watch strap, or another apparatus. The wearable component 202 is an apparatus that can attach the watch body 201 to the wrist of the user. The wearable device 100 is attached to the wrist of the user, so that an inertial sensor can collect motion data of the wrist of the user, to monitor motion of the wrist of the user and determine a posture of the user.

When the wearable device 100 starts to measure blood pressure, the wearable device 100 may control the wearable component 202 to shrink and then expand, to measure blood pressure of the user.

In some embodiments, a process of measuring blood pressure by the wearable device 100 may include: The wearable device 100 first inflates the wearable component 202 to temporarily block an upper-limb artery; then records, during slow deflation, a pressure value of the wearable component 202 and a pulse signal generated by a pulse; and finally determines blood pressure of the user based on the pressure value of the wearable component 202 and an amplitude or an envelope of the pulse signal. Blood flow causes lateral pressure to a vascular wall. A change in a magnitude of the lateral pressure causes slight vibration of the vascular wall. The pulse signal is a signal generated through the slight vibration of the vascular wall. Determining the blood pressure of the user based on the pressure value of the wearable component 202 and the amplitude or the envelope of the pulse signal is also referred to as an oscillometric method.

In another embodiment, a process of measuring blood pressure by the wearable device 100 may include: The wearable device 100 may gradually inflate the wearable component 202, so that an upper-limb artery changes from being gradually blocked to being completely blocked; record a pressure value of the wearable component 202 and a pulse signal generated by a pulse; then determine blood pressure of the user based on the pressure value of the wearable component 202 and an amplitude or an envelope of the pulse signal; and finally perform deflation. Blood flow causes lateral pressure to a vascular wall. A change in a magnitude of the lateral pressure causes slight vibration of the vascular wall. The pulse signal is a signal generated through the slight vibration of the vascular wall. Determining the blood pressure of the user based on the pressure value of the wearable component 202 and the amplitude or the envelope of the pulse signal is also referred to as an oscillometric method.

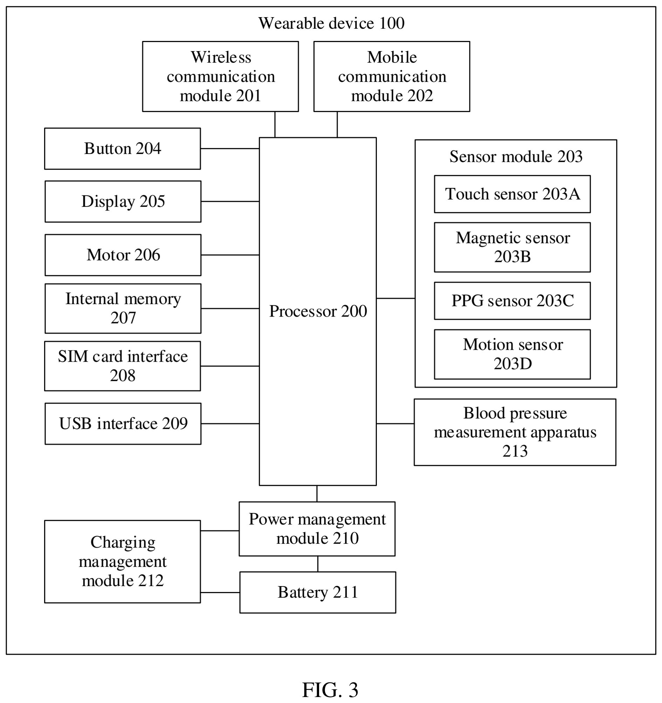

FIG. 3 is a diagram of a hardware structure of a wearable device 100.

As shown in FIG. 3, the wearable device may be a wristband, a watch, or another wearable device; or the wearable device 100 may be a non-wearable device like a wall-mounted blood pressure monitor. A specific type of the wearable device is not particularly limited in this embodiment of this application. In this embodiment of this application, only an example in which the wearable device 100 is a watch is used for description.

The wearable device 100 may include a processor 200, a wireless communication module 201, a mobile communication module 202, a sensor module 203, a button 204, a display 205, a motor 206, an internal memory 207, a SIM card interface 208, a USB interface 209, a power management module 210, a battery 211, a charging management module 212, and a blood pressure measurement apparatus 213. The sensor module 203 may include a touch sensor 203A, a magnetic sensor 203B, a photoplethysmography (photoplethysmography, PPG) sensor 203C, and a motion sensor 203D. A function of the airbag 213G is similar to a function of the wearable component 202.

It can be understood that the structure shown in this embodiment of the present invention does not constitute a specific limitation on the wearable device. In some other embodiments of this application, the wearable device may include more or fewer components than those shown in the figure, or some components may be combined, or some components may be split, or the components may be arranged differently. The components shown in the figure may be implemented by hardware, software, or a combination of software and hardware.

The processor 200 may include one or more processing units. For example, the processor 200 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent components, or may be integrated into one or more processors.

In some embodiments, the processor 200 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

In some embodiments, the processor 200 may alternatively be a micro processing unit (microcontroller unit, MCU).

The I2C interface is a two-way synchronous serial bus, and includes a serial data line (serial data line, SDA) and a serial clock line (serial clock line, SCL). In some embodiments, the processor 200 may include a plurality of groups of I2C buses. The processor 200 may be coupled to the touch sensor 203A, the power management module 210, and the like separately through different I2C bus interfaces. For example, the processor 200 may be coupled to the touch sensor 203A through the I2C interface, so that the processor 200 communicates with the touch sensor 203A through the I2C bus interface, to implement a touch function of the wearable device.

The I2S interface may be used for audio communication. The PCM interface may also be used for audio communication, and sampling, quantization, and encoding of an analog signal. The UART interface is a universal serial data bus, and is used for asynchronous communication. The bus may be a two-way communication bus. The bus converts to-be-transmitted data between serial communication and parallel communication. In some embodiments, the UART interface is usually configured to connect the processor 200 to the wireless communication module 201. For example, the processor 200 communicates with a Bluetooth module in the wireless communication module 201 through the UART interface, to implement a Bluetooth function.

The MIPI interface may be configured to connect the processor 200 to a peripheral component, for example, the display 205. The MIPI interface includes a camera serial interface (camera serial interface, CSI), a display serial interface (display serial interface, DSI), and the like. The processor 200 communicates with the display 205 through the DSI interface, to implement a display function of the wearable device.

The GPIO interface may be configured by software. The GPIO interface may be configured as a control signal or a data signal. The USB interface 209 is an interface that conforms to a USB standard specification, and may be specifically a mini USB interface, a micro USB interface, a USB Type-C interface, or the like. The USB interface 209 may be configured to connect to a charger to charge the wearable device, or may be configured to transmit data between the wearable device and a peripheral device.

It can be understood that an interface connection relationship between the modules in this embodiment of the present invention is merely an example for description, and does not constitute a limitation on a structure of the wearable device. In some other embodiments of this application, the wearable device may alternatively use an interface connection mode different from that in the foregoing embodiment, or use a combination of a plurality of interface connection modes.

The charging management module 212 is configured to receive a charging input from a charger. The charger may be a wireless charger or a wired charger. In some embodiments of wired charging, the charging management module 212 may receive a charging input from a wired charger through the USB interface 209. In some embodiments of wireless charging, the charging management module 212 may receive a wireless charging input through a wireless charging coil of the wearable device. When charging the battery 211, the charging management module 212 may further supply power to the wearable device through the power management module 210.

The power management module 210 is configured to connect to the battery 211, the charging management module 212, and the processor 200. The power management module 210 receives an input from the battery 211 and/or the charging management module 212, and supplies power to the processor 200, the internal memory 207, the display 205, the wireless communication module 201, and the like. The power management module 210 may be further configured to monitor parameters such as a battery capacity, a quantity of battery cycles, and a battery health status (electric leakage or impedance). In some other embodiments, the power management module 210 may alternatively be disposed in the processor 200. In some other embodiments, the power management module 210 and the charging management module 212 may alternatively be disposed in a same component.

A wireless communication function of the wearable device may be implemented by the mobile communication module 202, the wireless communication module 201, the modem processor, the baseband processor, and the like.

The mobile communication module 202 may provide a solution applied to the wearable device for wireless communication such as 2G/3G/4G/5G. The mobile communication module 202 may include at least one filter, a switch, a power amplifier, a low noise amplifier (low noise amplifier, LNA), and the like. The mobile communication module 202 may receive an electromagnetic wave through the antenna, perform processing such as filtering or amplification on the received electromagnetic wave, and transmit a processed electromagnetic wave to the modem processor for demodulation. In some embodiments, at least some functional modules of the mobile communication module 202 may be disposed in the processor 200. In some embodiments, at least some functional modules of the mobile communication module 202 may be disposed in a same component as at least some modules of the processor 200.

The wireless communication module 201 may provide a solution applied to the wearable device for wireless communication such as a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, or an infrared (infrared, IR) technology. The wireless communication module 201 may be one or more components integrating at least one communication processor module. The wireless communication module 201 receives an electromagnetic wave through the antenna, performs frequency modulation and filtering on an electromagnetic wave signal, and sends a processed signal to the processor 200. The wireless communication module 201 may further receive a to-be-sent signal from the processor 200, perform frequency modulation and amplification on the signal, and convert a processed signal into an electromagnetic wave for radiation through the antenna.

The button 204 includes a power button, a volume button, and the like. The button 204 may be a mechanical button or a touch button. The wearable device may receive an input on the button, and generate a button signal input related to a user setting and function control of the wearable device.

The display 205 is configured to display an image, a video, or the like. The display 205 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flex light-emitting diode, FLED), a quantum dot light-emitting diode (quantum dot light-emitting diode, QLED), or the like. In some embodiments, the wearable device may include one or N displays 205, where N is a positive integer greater than 1.

The motor 206 may generate a vibration prompt. The motor 206 may be configured to produce an incoming call vibration prompt or a touch vibration feedback. For example, touch operations performed on different applications (for example, photographing and audio playing) may correspond to different vibration feedback effects. The motor 206 may also correspond to different vibration feedback effects for touch operations performed on different areas of the display 205.

The internal memory 207 may include one or more random access memories (random access memory, RAM) and one or more non-volatile memories (non-volatile memory, NVM).

The random access memory may include a static random access memory (static random access memory, SRAM), a dynamic random access memory (dynamic random access memory, DRAM), a synchronous dynamic random access memory (synchronous dynamic random access memory, SDRAM), a double data rate synchronous dynamic random access memory (double data rate synchronous dynamic random access memory, DDR SDRAM, for example, a fifth-generation DDR SDRAM is usually referred to as a DDR5 SDRAM), and the like.

The non-volatile memory may include a magnetic disk storage device and a flash memory (flash memory). The flash memory may include a NOR flash, a NAND flash, a 3D NAND flash, and the like through division according to an operation principle; may include a single-level cell (single-level cell, SLC), a multi-level cell (multi-level cell, MLC), a triple-level cell (triple-level cell, TLC), a quad-level cell (quad-level cell, QLC), and the like through division based on a potential order of a storage unit; or may include a universal flash storage (English: universal flash storage, UFS), an embedded multimedia card (embedded multimedia card, eMMC), and the like through division based on storage specifications. The processor 200 may directly perform a read or write operation on the random access memory. The random access memory may be configured to store executable programs (for example, machine instructions) of an operating system or another running program, and may be further configured to store data of a user and an application, and the like. The non-volatile memory may also store an executable program, data of a user and an application, and the like, which may be pre-loaded to the random access memory for the processor 200 to directly perform a read or write operation.

The SIM card interface 208 is used for connecting a SIM card. The SIM card may be inserted into the SIM card interface 208 or removed from the SIM card interface 208, to implement contact with or separation from the wearable device. The wearable device may support one or N SIM card interfaces, where N is a positive integer greater than 1. The SIM card interface 208 may support a nano-SIM card, a micro-SIM card, a SIM card, and the like. A plurality of cards may be inserted in a same SIM card interface 208 at the same time. The plurality of cards may be of a same type or different types. The SIM card interface 208 may also be compatible with different types of SIM cards. The SIM card interface 208 may also be compatible with an external memory card. The wearable device interacts with a network through the SIM card, to implement functions such as calling and data communication. In some embodiments, an eSIM, namely, an embedded SIM card, is used for the wearable device. The eSIM card may be embedded into the wearable device, and cannot be separated from the wearable device.

In some embodiments, the wearable device 100 may alternatively not include the SIM card interface 208.

The touch sensor 203A is also referred to as a "touch device". The touch sensor 203A may be disposed in the display 205. The touch sensor 203A and the display 205 constitute a touchscreen, which is also referred to as a "touch screen". The touch sensor 203A is configured to detect a touch operation performed on or near the touch sensor. The touch sensor may transmit the detected touch operation to the application processor to determine a type of a touch event. The display 205 may provide a visual output related to the touch operation. In some other embodiments, the touch sensor 203A may alternatively be disposed on a surface of the wearable device at a position different from a position of the display 205.

The magnetic sensor 203B includes a Hall effect sensor.

The PPG sensor 203C is configured to obtain health data of the user based on a PPG signal collected by the PPG sensor 203C. The health data of the user includes but is not limited to a heart rate, blood oxygen, a respiratory rate, blood oxygen saturation (SaO₂), and the like.

The motion sensor 203D includes but is not limited to an acceleration sensor and an angular velocity sensor. The motion sensor 203D may be configured to collect motion data, determine a motion status of the user based on the motion data, and then determine, based on the motion status of the user, whether the user is in a sleep state.

It should be noted that the sensor module 203 may further include an infrared sensor and the like.

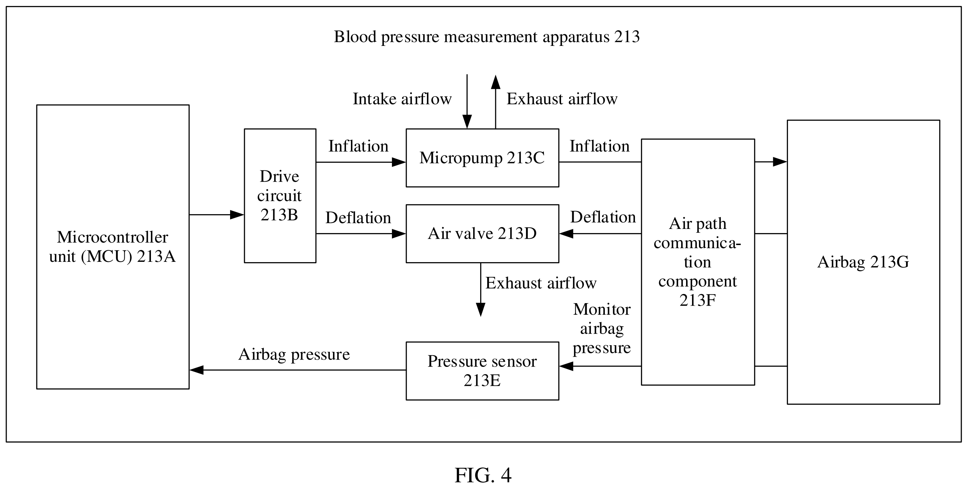

The blood pressure measurement apparatus 213 may be configured to measure blood pressure. For descriptions of the blood pressure measurement apparatus 213, refer to descriptions in an embodiment of FIG. 4.

It should be noted that the wearable device 100 may include more or fewer hardware structures than those in FIG. 3. The embodiment of FIG. 3 is merely an example. This is not limited in this application.

FIG. 4 is a diagram of a composition structure of a blood pressure measurement apparatus 213 according to this application.

As shown in FIG. 4, the blood pressure measurement apparatus 213 may include but is not limited to a microcontroller unit (microcontroller unit, MCU) 213A, a drive circuit 213B, a micropump 213C, an air valve 213D, a pressure sensor 213E, an air path communication component 213F, and an airbag 213G.

The drive circuit 213B is connected to the microcontroller unit 213A, and the pressure sensor 213E is also connected to the microcontroller unit 213A. The microcontroller unit 213A is configured to control, based on airbag pressure obtained by the pressure sensor 213E, the drive circuit 213B to adjust a voltage of the drive circuit 213B, so that the drive circuit 213B can control the micropump 213C to inflate the airbag 213G and control the air valve 213D to exhaust air in the airbag 213G.

The pressure sensor 213E is configured to measure pressure. In some embodiments of this application, the wearable device measures pressure in the airbag 213G through the pressure sensor 213E. In some embodiments of this application, some components of the pressure sensor 213E are located in the airbag 213G, and are configured to sense pressure in the airbag 213G.

In some embodiments of this application, the pressure sensor 213E and the airbag 213G may be connected through the air path communication component 213F.

The micropump 213C and the air valve 213D are connected to the drive circuit 213B. The drive circuit 213B may control the micropump 213C to inflate the airbag 213G, and the drive circuit 213B may also control the air valve 213D to exhaust air in the airbag 213G.

The micropump 213C is configured to perform inflation and deflation.

In some embodiments of this application, the wearable device 100 inflates the airbag 213G through the micropump 213C. The micropump 213C and the airbag 213G may be connected through the air path communication component 213F. The airbag 213G is configured to press against a blood vessel of a user, and has a function similar to that of a cuff in a conventional blood pressure measurement apparatus.

In some embodiments of this application, the wearable device 100 may exhaust air in the airbag 213G through the air valve 213D. The air valve 213D and the airbag 213G may be connected through the air path communication component 213F. In another embodiment, the wearable device 100 may alternatively not include the air valve 213D. This is not limited in this application.

It should be noted that the air path communication component 213F may be an independent component, or the air path communication component 213F may be an air path formed by combining other hardware modules, or the air path communication component 213F may be a part of another component, for example, a part of the micropump 213C, or a part of the airbag 213G.

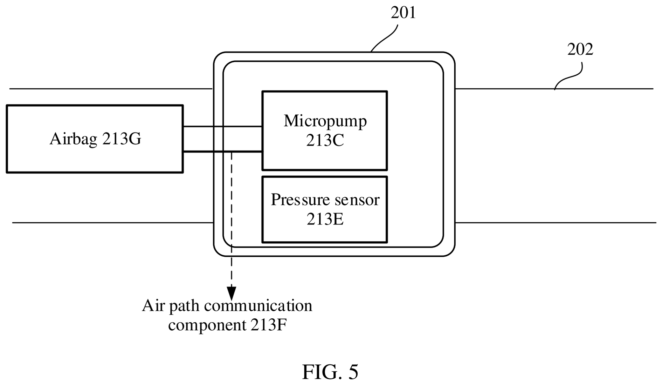

As shown in FIG. 5, when the wearable device 100 is a watch, the airbag 213G is attached to a side, close to a body, of the wearable component 202. The micropump 213C is connected to the airbag 213G through the air path communication component 213F. The airbag 213G may be attached only to one side of the wearable component 202, and the side of the wearable component 202 may be located above an artery position on a wrist of the user, for example, above a radial artery position.

The airbag 213G may be located in a watch body of a smartwatch. The airbag 213G may be connected to a buckle of a watch strap, and the airbag 213G is connected to a watch face through an air vent cover. Correspondingly, the airbag 213G may be separated from the watch strap, or may be separated from the watch face.

In some embodiments of this application, the wearable device 100 is equipped with a magnetic sensor 203B, and may determine, through the magnetic sensor 203B, whether the airbag 213G on the wearable device 100 is removed. For example, the airbag 213G or the watch strap connected to the airbag 213G may be equipped with a magnet. The wearable device may determine, through the magnetic sensor, a magnetic flux generated by the airbag 213G or the magnet on the airbag 213G, to determine whether the airbag 213G on the wearable device is removed.

It should be noted that the blood pressure measurement apparatus 213 may include more or fewer hardware structures than those in FIG. 4. The embodiment of FIG. 4 is merely an example. This is not limited in this application.

In some embodiments of this application, the wearable device 100 may be provided with N constant pressure control points. When starting to measure blood pressure, the wearable device 100 may inflate the airbag 213G at an air inflation rate v1. In addition, the micropump 213C slightly releases air. For example, an air release rate of the airbag 213G is v2. In this case, it can be learned that a pressure increase rate of pressure in the airbag 213G is (v1-v2), so that the pressure in the airbag 213G uniformly increases at the pressure increase rate (v1-v2). When a first constant pressure control point P1 is reached, the wearable device 100 may control the pressure in the airbag 213G to be P1 and remain unchanged for first duration. After the first duration elapses, the wearable device 100 may continue to inflate the airbag 213G at the air inflation rate v1, so that the pressure in the airbag 213G uniformly increases at the pressure increase rate (v1-v2). When a second constant pressure control point P2 is reached, the wearable device 100 may control the pressure in the airbag 213G to be P2 and remain unchanged for second duration. P2 is greater than P1. The second duration may be equal to the first duration, or the second duration may be different from the first duration. In this manner, the wearable device 100 may obtain N linear pressure increase periods and N constant pressure control periods, and obtain peripheral arterial pulse wave signals within the N linear pressure increase periods and peripheral arterial pulse wave signals within the N constant pressure control periods.

The wearable device 100 may obtain a peripheral blood pressure measurement value based on the peripheral arterial pulse wave signals within the N linear pressure increase periods and an airbag pressure waveform graph within the N linear pressure increase periods.

The wearable device 100 may also obtain a central arterial pulse wave signal based on the peripheral arterial pulse wave signals within the N constant pressure control periods. The wearable device 100 may perform feature analysis on the central arterial pulse wave signal to obtain a cardiovascular disease risk. The cardiovascular disease risk includes but is not limited to a peripheral resistance risk, an arteriosclerosis risk, and the like.

In some embodiments, the wearable device may further obtain a central arterial blood pressure value based on the peripheral blood pressure measurement value and the central arterial pulse wave signal, and the central arterial blood pressure value can better indicate an actual blood pressure value of the user.
**1. The wearable device 100 obtains N linear pressure increase periods and N constant pressure control periods.**

This application provides two methods for obtaining the N linear pressure increase periods and the N constant pressure control periods. In one method, the wearable device 100 may adjust an air inflation rate of the micropump 213C to obtain the N linear pressure increase periods and the N constant pressure control periods. In the other method, the wearable device 100 may keep an air inflation rate of the micropump 213C fixed, and adjust an air release rate of the air valve 213D to obtain the N linear pressure increase periods and the N constant pressure control periods.

The following separately describes in detail the foregoing two methods for obtaining the N linear pressure increase periods and the N constant pressure control periods.

**Method 1: The wearable device 100 adjusts the air inflation rate of the micropump 213C to obtain the N linear pressure increase periods and the N constant pressure control periods.**

In the method 1, the wearable device 100 inflates and deflates the airbag 213G through the micropump 213C. The wearable device 100 may control the micropump 213C to inflate the airbag 213G, or the wearable device 100 may control the micropump 213C to exhaust air in the airbag 213G. An air release rate of the micropump 213C is fixed. For example, the air release rate of the micropump 213C is v2.

The wearable device 100 may be preconfigured with N constant pressure control points, for example, four constant pressure control points. The constant pressure control points are sequentially 30 mmHg, 60 mmHg, 90 mmHg, and 120 mmHg. In this case, the wearable device 100 may separately obtain five linear pressure increase periods and four constant pressure control periods.

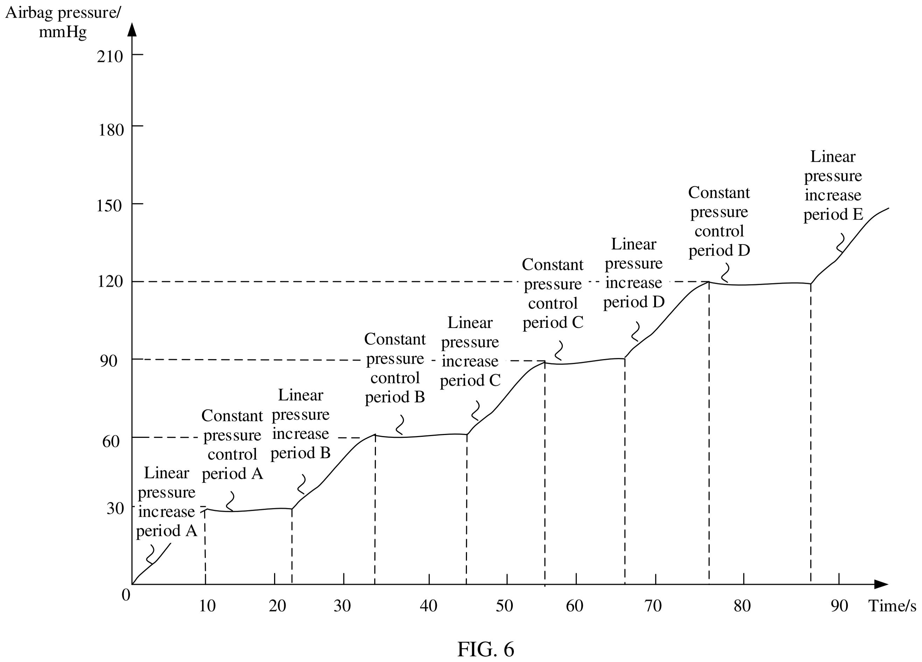

FIG. 6 is a diagram of the five linear pressure increase periods and the four constant pressure control periods obtained that are obtained by the wearable device 100.

As shown in FIG. 6, the five linear pressure increase periods may include but are not limited to a linear pressure increase period A, a linear pressure increase period B, a linear pressure increase period C, a linear pressure increase period D, and a linear pressure increase period E. The four constant pressure control periods may include but are not limited to a constant pressure control period A, a constant pressure control period B, a constant pressure control period C, and a constant pressure control period D.

That the wearable device 100 obtains the linear pressure increase period A may include: When blood pressure measurement starts, for example, at a moment 0, pressure in the airbag 213G is close to 0 mmHg. Before the pressure in the airbag 213G reaches the first constant pressure control point 30 mmHg, the wearable device 100 may control a duty cycle of the drive circuit 213B to be a first duty cycle. In response to the duty cycle of the drive circuit 213B being the first duty cycle, the drive circuit 213B may control the micropump 213C to inflate the airbag 213G at an air inflation rate v1. In addition, the micropump 213C slightly releases air. For example, an air release rate of the airbag 213G is v2. In this case, it can be learned that a pressure increase rate of the pressure in the airbag 213G is (v1-v2). For example, as shown in FIG. 6, at 0s to 10s, the pressure in the airbag 213G uniformly increases to 30 mmHg. In this case, an average pressure increase rate of the pressure in the airbag 213G is 3 mmHg/s.

It should be noted that, during the linear pressure increase period A, the pressure sensor 213E also obtains a peripheral arterial pulse wave signal of the user in real time.

It should be noted that a pressure increase rate of the pressure in the airbag 213G within a linear pressure increase period may change within a specific range. For example, the pressure increase rate of the pressure within the linear pressure increase period A may fluctuate from 2.8 mmHg/s to 3.2 mmHg/s, and may not be fixed.

That the wearable device 100 obtains the constant pressure control period A may include: The pressure sensor 213E detects that the pressure in the airbag 213G reaches 30 mmHg, to be specific, the pressure in the airbag 213G reaches the first constant pressure control point 30 mmHg. To keep the pressure in the airbag 213G at 30 mmHg, the wearable device 100 may control the duty cycle of the drive circuit 213B to be a second duty cycle. In response to the duty cycle of the drive circuit 213B being the second duty cycle, the drive circuit 213B may control the micropump 213C to inflate the airbag 213G at an air inflation rate v2. To be specific, the air inflation rate of the airbag 213G is the same as the air release rate of the airbag 213G, so that the pressure in the airbag 213G remains unchanged and is fixed at 30 mmHg.

At 11s to 22s, the pressure in the airbag 213G remains at 30 mmHg. In this case, the average pressure increase rate of the pressure in the airbag 213G is 0.

Optionally, retention time within the linear pressure increase period A may be set to 12s.

It should be noted that, within a constant pressure control period, the pressure in the airbag may remain unchanged, or may fluctuate within a specific range. For example, when pressure at a constant pressure control point is 30 mmHg, the pressure in the airbag within the constant pressure control period may fluctuate within a range of 25 mmHg to 35 mmHg.

That the wearable device 100 obtains the linear pressure increase period B may include: After the pressure sensor 213E detects that the pressure in the airbag 213G has remained at 30 mmHg for 12s, the wearable device 100 may control the duty cycle of the drive circuit 213B to be the first duty cycle. In response to the duty cycle of the drive circuit 213B being the first duty cycle, the drive circuit 213B may control the micropump 213C to inflate the airbag 213G at the air inflation rate v1. In addition, the micropump 213C slightly releases air. For example, the air release rate of the airbag 213G is v2. In this case, it can be learned that the pressure increase rate of the pressure in the airbag 213G is (v1-v2).

For example, as shown in FIG. 6, at 23s to 32s, the pressure in the airbag 213G uniformly increases to 60 mmHg. In this case, the average pressure increase rate of the pressure in the airbag 213G is 3 mmHg/s. The average pressure increase rate within the linear pressure increase period B is the same as the average pressure increase rate within the linear pressure increase period A.

It should be noted that, during the linear pressure increase period B, the pressure sensor 213E also obtains a peripheral arterial pulse wave signal of the user in real time.

That the wearable device 100 obtains the constant pressure control period B may include: The pressure sensor 213E detects that the pressure in the airbag 213G reaches 60 mmHg, to be specific, the pressure in the airbag 213G reaches the second constant pressure control point 60 mmHg. To keep the pressure in the airbag 213G at 60 mmHg, the wearable device 100 may control the duty cycle of the drive circuit 213B to be the second duty cycle. In response to the duty cycle of the drive circuit 213B being the second duty cycle, the drive circuit 213B may control the micropump 213C to inflate the airbag 213G at the air inflation rate v2. To be specific, the air inflation rate of the airbag 213G is the same as the air release rate of the airbag 213G, so that the pressure in the airbag 213G remains unchanged and is fixed at 60 mmHg.

For example, as shown in FIG. 6, at 33s to 44s, the pressure in the airbag 213G remains at 60 mmHg. In this case, the average pressure increase rate of the pressure in the airbag 213G is 0.

Optionally, retention time within the constant pressure control period B may be set to 12s.

It should be noted that, during the constant pressure control period B, the pressure sensor 213E also obtains a peripheral arterial pulse wave signal of the user in real time. Within the constant pressure control period B, the pressure in the airbag 213G is fixed. Therefore, the peripheral arterial pulse wave signal obtained by the pressure sensor 213E has fewer lost frames than the peripheral arterial pulse wave signal obtained by the pressure sensor 213E within the linear pressure increase period B, and has good effect.

That the wearable device 100 obtains the linear pressure increase period C may include: After the pressure sensor 213E detects that the pressure in the airbag 213G has remained at 90 mmHg for 12s, the wearable device 100 may control the duty cycle of the drive circuit 213B to be the first duty cycle. In response to the duty cycle of the drive circuit 213B being the first duty cycle, the drive circuit 213B may control the micropump 213C to inflate the airbag 213G at the air inflation rate v1. In addition, the micropump 213C slightly releases air. For example, the air release rate of the airbag 213G is v2. In this case, it can be learned that the pressure increase rate of the pressure in the airbag 213G is (v1-v2).

For example, as shown in FIG. 6, at 45s to 54s, the pressure in the airbag 213G uniformly increases to 90 mmHg. In this case, the average pressure increase rate of the pressure in the airbag 213G is 3 mmHg/s. The average pressure increase rate within the linear pressure increase period C is the same as the average pressure increase rate within the linear pressure increase period B.

It should be noted that, during the linear pressure increase period C, the pressure sensor 213E also obtains a peripheral arterial pulse wave signal of the user in real time.

That the wearable device 100 obtains the constant pressure control period C may include: The pressure sensor 213E detects that the pressure in the airbag 213G reaches 90 mmHg, to be specific, the pressure in the airbag 213G reaches the third constant pressure control point 90 mmHg. To keep the pressure in the airbag 213G at 90 mmHg, the wearable device 100 may control the duty cycle of the drive circuit 213B to be the second duty cycle. In response to the duty cycle of the drive circuit 213B being the second duty cycle, the drive circuit 213B may control the micropump 213C to inflate the airbag 213G at the air inflation rate v2. To be specific, the air inflation rate of the airbag 213G is the same as the air release rate of the airbag 213G, so that the pressure in the airbag 213G remains unchanged and is fixed at 90 mmHg.

For example, as shown inFIG. 6, at 55s to 66s, the pressure in the airbag 213G remains at 90 mmHg. In this case, the average pressure increase rate of the pressure in the airbag 213G is 0.

Optionally, retention time within the constant pressure control period C may be set to 12s.

It should be noted that, during the constant pressure control period C, the pressure sensor 213E also obtains a peripheral arterial pulse wave signal of the user in real time. Within the constant pressure control period C, the pressure in the airbag 213G is fixed. Therefore, the peripheral arterial pulse wave signal obtained by the pressure sensor 213E has fewer lost frames than the peripheral arterial pulse wave signal obtained by the pressure sensor 213E within the linear pressure increase period C, and has good effect.

That the wearable device 100 obtains the linear pressure increase period D may include: After the pressure sensor 213E detects that the pressure in the airbag 213G has remained at 90 mmHg for 12s, the wearable device 100 may control the duty cycle of the drive circuit 213B to be the first duty cycle. In response to the duty cycle of the drive circuit 213B being the first duty cycle, the drive circuit 213B may control the micropump 213C to inflate the airbag 213G at the air inflation rate v1. In addition, the micropump 213C slightly releases air. For example, the air release rate of the airbag 213G is v2. In this case, it can be learned that the pressure increase rate of the pressure in the airbag 213G is (v1-v2).

For example, as shown in FIG. 6, at 67s to 76s, the pressure in the airbag 213G uniformly increases to 120 mmHg. In this case, the average pressure increase rate of the pressure in the airbag 213G is 3 mmHg/s. The average pressure increase rate within the linear pressure increase period D is the same as the average pressure increase rate within the linear pressure increase period C.

It should be noted that, during the linear pressure increase period D, the pressure sensor 213E also obtains a peripheral arterial pulse wave signal of the user in real time.

That the wearable device 100 obtains the constant pressure control period D may include: The pressure sensor 213E detects that the pressure in the airbag 213G reaches 120 mmHg, to be specific, the pressure in the airbag 213G reaches the fourth constant pressure control point 120 mmHg. To keep the pressure in the airbag 213G at 120 mmHg, the wearable device 100 may control the duty cycle of the drive circuit 213B to be the second duty cycle. In response to the duty cycle of the drive circuit 213B being the second duty cycle, the drive circuit 213B may control the micropump 213C to inflate the airbag 213G at the air inflation rate v2. To be specific, the air inflation rate of the airbag 213G is the same as the air release rate of the airbag 213G, so that the pressure in the airbag 213G remains unchanged and is fixed at 120 mmHg.

For example, as shown in FIG. 6, at 77s to 88s, the pressure in the airbag 213G remains at 120 mmHg. In this case, the average pressure increase rate of the pressure in the airbag 213G is 0.

Optionally, retention time within the constant pressure control period D may be set to 12s.

It should be noted that, during the constant pressure control period D, the pressure sensor 213E also obtains a peripheral arterial pulse wave signal of the user in real time. Within the constant pressure control period D, the pressure in the airbag 213G is fixed. Therefore, the peripheral arterial pulse wave signal obtained by the pressure sensor 213E has fewer lost frames than the peripheral arterial pulse wave signal obtained by the pressure sensor 213E within the linear pressure increase period D, and has good effect.

That the wearable device 100 obtains the linear pressure increase period E may include: After the pressure sensor 213E detects that the pressure in the airbag 213G has remained at 120 mmHg for 12s, the wearable device 100 may control the duty cycle of the drive circuit 213B to be the first duty cycle. In response to the duty cycle of the drive circuit 213B being the first duty cycle, the drive circuit 213B may control the micropump 213C to inflate the airbag 213G at the air inflation rate v1. In addition, the micropump 213C slightly releases air. For example, the air release rate of the airbag 213G is v2. In this case, it can be learned that the pressure increase rate of the pressure in the airbag 213G is (v1-v2).

For example, as shown in FIG. 6, at 89s and later, the pressure in the airbag 213G uniformly increases to a maximum value, and the average pressure increase rate of the pressure in the airbag 213G is 3 mmHg/s. The average pressure increase rate within the linear pressure increase period E is the same as the average pressure increase rate within the linear pressure increase period D.

It should be noted that, during the linear pressure increase period E, the pressure sensor 213E also obtains a peripheral arterial pulse wave signal of the user in real time.

The foregoing steps are repeated until the micropump 213C stops inflating the airbag 213G, and blood pressure measurement ends.

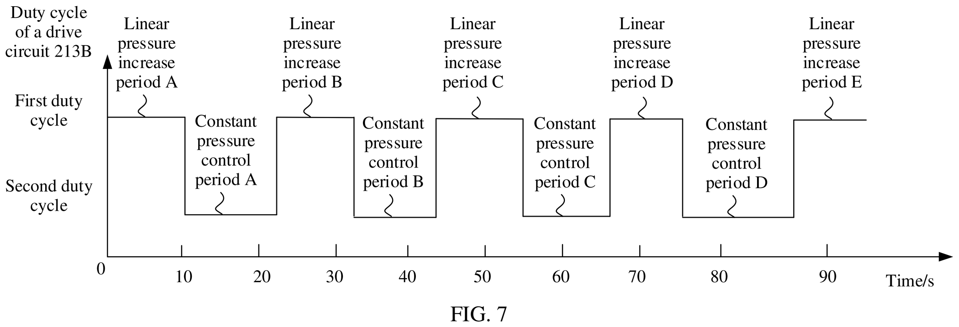

FIG. 7 is a diagram of duty cycles corresponding to the drive circuit 213B within linear pressure increase periods and constant pressure control periods. All of duty cycles of the drive circuit 213B within the linear pressure increase period A, the linear pressure increase period B, the linear pressure increase period C, the linear pressure increase period D, and the linear pressure increase period E are the first duty cycle. All of duty cycles of the drive circuit 213B within the constant pressure control period A, the constant pressure control period B, the constant pressure control period C, and the constant pressure control period D are the second duty cycle. The first duty cycle is greater than the second duty cycle. To be specific, in the method 1, duty cycles of the drive circuit 213B within the linear pressure increase periods are the same, and duty cycles of the drive circuit 213B within the constant pressure control periods are the same. However, the duty cycles of the drive circuit 213B within the linear pressure increase periods are greater than the duty cycles of the drive circuit 213B within the constant pressure control periods.

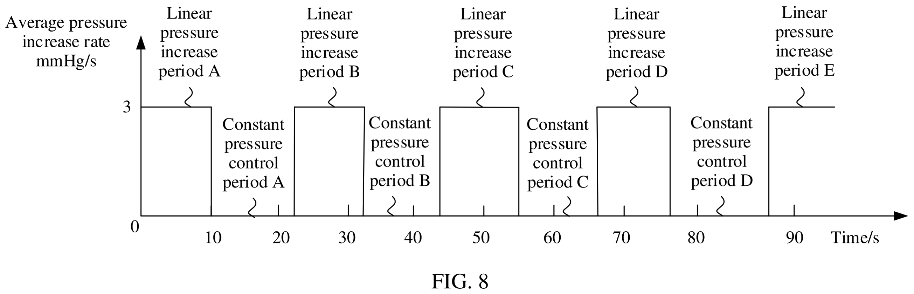

FIG. 8 is a diagram of average pressure increase rates of the airbag 213G within linear pressure increase periods and constant pressure control periods. All of average pressure increase rates of the airbag 213G within the linear pressure increase period A, the linear pressure increase period B, the linear pressure increase period C, the linear pressure increase period D, and the linear pressure increase period E are 3 mmHg/s. All of average pressure increase rates of the airbag 213G within the constant pressure control period A, the constant pressure control period B, the constant pressure control period C, and the constant pressure control period D are 0. To be specific, in the method 1, average pressure increase rates of the airbag 213G within linear pressure increase periods are the same, average pressure increase rates of the airbag 213G within constant pressure control periods are close to 0, and the average pressure increase rates of the airbag 213G within the constant pressure control periods are fixed.

It should be noted that, in addition to the five linear pressure increase periods and the four constant pressure control periods, more or fewer linear pressure increase periods and constant pressure control periods may alternatively be set for the wearable device 100. Details are not described herein in this application.

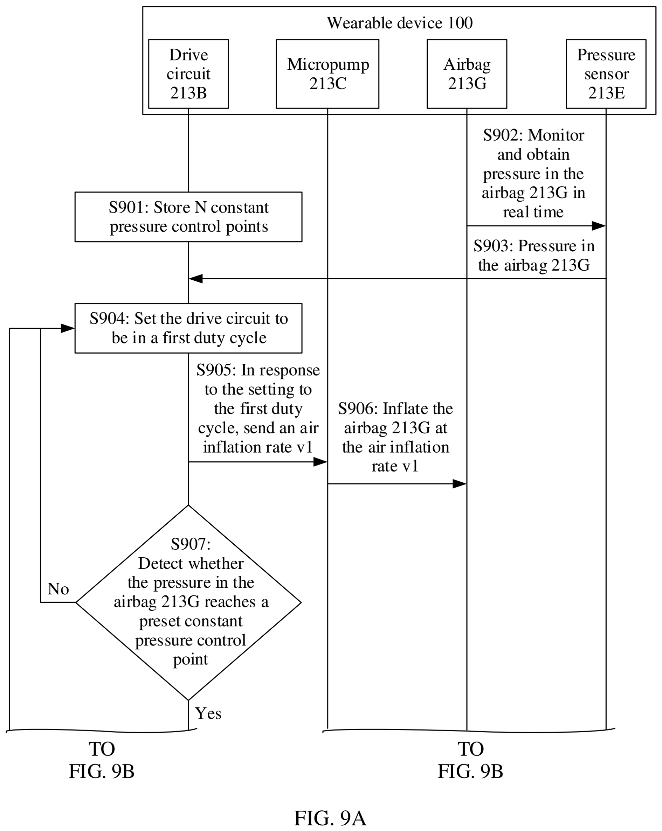

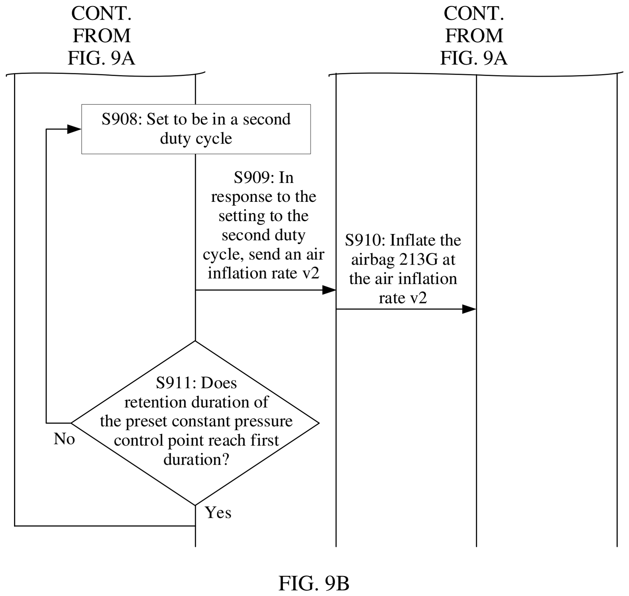

**FIG. 9A** **and** **FIG. 9B** **are a schematic flowchart of a method for obtaining N linear pressure increase periods and N constant pressure control periods by a wearable device 100.**

As shown in FIG. 9A and FIG. 9B, the wearable device 100 includes a drive circuit 213B, a micropump 213C, an airbag 213G, and a pressure sensor 213E.

In some embodiments, the wearable device 100 may further include an air valve 213D. During blood pressure measurement, the air valve 213D remains in a closed state.

S901: The drive circuit 213B stores N constant pressure control points.

The drive circuit 213B stores the N constant pressure control points, where N is a positive integer greater than or equal to 1. When pressure in the airbag 213G reaches a constant pressure control point, the drive circuit 213B may adjust an air inflation rate of the micropump 213C to keep the pressure in the airbag 213G unchanged, so that the pressure sensor 213E can obtain a peripheral arterial pulse wave signal within a constant pressure control period.

For example, the N constant pressure control points may be sequentially 30 mmHg, 60 mmHg, 90 mmHg, and 120 mmHg.

S902: The pressure sensor 213E monitors and obtains the pressure in the airbag 213G in real time.

S903: The pressure sensor 213E sends the obtained pressure in the airbag 213G to the drive circuit 213B.

The pressure sensor 213E may be connected to the airbag 213G. For example, the pressure sensor 213E and the airbag 213G may be connected through an air path communication component 213F.

The pressure sensor 213E may monitor the pressure in the airbag 213G in real time, and send the obtained pressure in the airbag 213G to the drive circuit 213B.

S904: The drive circuit 213B sets the drive circuit to be in a first duty cycle.

S905: In response to the setting to the first duty cycle, the drive circuit 213B sends an air inflation rate v1 to the micropump 213C.

S906: The micropump 213C inflates the airbag 213G at the air inflation rate v1.

The drive circuit 213B receives an instruction and starts to measure blood pressure. When starting to measure blood pressure, the drive circuit 213B sets the drive circuit to be in the first duty cycle. In response to the setting to the first duty cycle, the drive circuit 213B sends the air inflation rate v1 to the micropump 213C, so that the micropump 213C inflates the airbag 213G at the air inflation rate v1. In addition, the micropump 213C slightly releases air. For example, an air release rate of the airbag 213G is v2. In this case, it can be learned that a pressure increase rate of the pressure in the airbag 213G is (v1-v2), so that the pressure in the airbag 213G uniformly increases at the pressure increase rate (v1-v2).

S907: The drive circuit 213B further needs to detect whether the pressure in the airbag 213G reaches a preset constant pressure control point.

The preset constant pressure control point may be any one of the N constant pressure control points. For example, the preset constant pressure control point may be 30 mmHg, 60 mmHg, 90 mmHg, or 120 mmHg.

The pressure sensor 213E may periodically or aperiodically send the pressure in the airbag 213G to the drive circuit 213B, and the drive circuit 213B needs to detect whether the pressure in the airbag 213G reaches the preset constant pressure control point.

In this way, when the pressure in the airbag 213G reaches the preset constant pressure control point, the drive circuit 213B may adjust the air inflation rate of the micropump 213C to keep the pressure in the airbag 213G constant, to obtain a peripheral arterial pulse wave signal within a constant pressure control period.

When the pressure in the airbag 213G does not reach the preset constant pressure control point, the drive circuit 213B may not adjust the air inflation rate of the micropump 213C, so that the pressure in the airbag 213G uniformly increases at the pressure increase rate (v1-v2).

When the pressure in the airbag 213G reaches the preset constant pressure control point, S908 is performed.

When the pressure in the airbag 213G does not reach the preset constant pressure control point, S904 is performed. Alternatively, when the pressure in the airbag 213G does not reach the preset constant pressure control point, S904 is not performed, and the micropump 213C still keeps inflating the airbag 213G at the air inflation rate v1.

S908: The drive circuit 213B sets the drive circuit to be in a second duty cycle.

S909: In response to the setting to the second duty cycle, the drive circuit 213B sends an air inflation rate v2 to the micropump 213C.

S910: The micropump 213C inflates the airbag 213G at the air inflation rate v2.

When the pressure in the airbag 213G reaches the preset constant pressure control point, the drive circuit 213B sets the drive circuit to be in the second duty cycle. The second duty cycle is less than the first duty cycle. A smaller duty cycle indicates a smaller air inflation rate.

In response to the setting to the second duty cycle, the drive circuit 213B sends the air inflation rate v2 to the micropump 213C, so that the micropump 213C inflates the airbag 213G at the air inflation rate v2. In addition, the micropump 213C slightly releases air. For example, the air release rate of the airbag 213G is v2. In this case, it can be learned that the pressure increase rate of the pressure in the airbag 213G is 0, and the pressure in the airbag 213G remains unchanged.

In this way, when the pressure in the airbag 213G reaches the preset constant pressure control point, the micropump 213C reduces the air inflation rate, so that the air inflation rate of the airbag 213G is equal to the air release rate of the airbag 213G. The pressure sensor 213E may obtain a peripheral arterial pulse wave signal within a constant pressure control period.

S911: The drive circuit 213B needs to determine whether retention duration of the preset constant pressure control point reaches first duration.

After the pressure in the airbag 213G reaches the preset constant pressure control point and the micropump 213C reduces the air inflation rate, the micropump 213C needs to determine whether the retention duration of the preset constant pressure control point reaches the first duration.

When the retention duration of the preset constant pressure control point reaches the first duration, S904 is performed. To be specific, the drive circuit 213B sets the drive circuit to be in the first duty cycle again, and the drive circuit 213B sends the air inflation rate v1 to the micropump 213C, so that the micropump 213C inflates the airbag 213G at the air inflation rate v1. In addition, the micropump 213C slightly releases air. For example, the air release rate of the airbag 213G is v2. In this case, it can be learned that the pressure increase rate of the pressure in the airbag 213G is (v1-v2), so that the pressure in the airbag 213G uniformly increases at the pressure increase rate (v1-v2).

When the retention duration of the preset constant pressure control point does not reach the first duration, S908 is performed. Alternatively, when the pressure in the airbag 213G does not reach the preset constant pressure control point, S908 is not performed. To be specific, the micropump 213C continues to inflate the airbag 213G at the air inflation rate v2, so that the pressure in the airbag 213G continues to remain constant.

In some embodiments, after blood pressure measurement is completed and the micropump 213C stops inflating the airbag 213G, the wearable device 100 may open the air valve 213D to increase the air release rate of the airbag 213G.

**Method 2: The wearable device 100 keeps the air inflation rate of the micropump 213C unchanged, and adjusts the air release rate of the air valve 213D to obtain the N linear pressure increase periods and the N constant pressure control periods.**

In the method 2, the wearable device 100 inflates and deflates the airbag 213G through the micropump 213C. The wearable device 100 may control the micropump 213C to inflate the airbag 213G, or the wearable device 100 may control the micropump 213C and the air valve 213D to exhaust air in the airbag 213G. An air release rate of the micropump 213C is fixed. For example, the air release rate of the micropump 213C is v2. The air release rate of the air valve 213D is adjustable.

The wearable device 100 may be preconfigured with N constant pressure control points, for example, four constant pressure control points. The constant pressure control points are sequentially 30 mmHg, 60 mmHg, 90 mmHg, and 120 mmHg. In this case, the wearable device 100 may separately obtain five linear pressure increase periods and four constant pressure control periods.

Refer to FIG. 6. The wearable device 100 may obtain five linear pressure increase periods and four constant pressure control periods.

As shown in FIG. 6, the five linear pressure increase periods may include but are not limited to a linear pressure increase period A, a linear pressure increase period B, a linear pressure increase period C, a linear pressure increase period D, and a linear pressure increase period E. The four constant pressure control periods may include but are not limited to a constant pressure control period A, a constant pressure control period B, a constant pressure control period C, and a constant pressure control period D.

That the wearable device 100 obtains the linear pressure increase period A may include: When blood pressure measurement starts, for example, at a moment 0, pressure in the airbag 213G is close to 0 mmHg. Before the pressure in the airbag 213G reaches the first constant pressure control point 30 mmHg, the wearable device 100 may control a duty cycle of the drive circuit 213B to be a first duty cycle, and control the air valve 213D to be closed. In response to the duty cycle of the drive circuit 213B being the first duty cycle, the drive circuit 213B may control the micropump 213C to inflate the airbag 213G at an air inflation rate v1. In addition, the micropump 213C slightly releases air. For example, an air release rate of the airbag 213G is v2. In this case, it can be learned that a pressure increase rate of the pressure in the airbag 213G is (v1-v2). For example, as shown in FIG. 6, at 0s to 10s, the pressure in the airbag 213G uniformly increases to 30 mmHg. In this case, an average pressure increase rate of the pressure in the airbag 213G is 3 mmHg/s.

It should be noted that, during the linear pressure increase period A, the pressure sensor 213E also obtains a peripheral arterial pulse wave signal of the user in real time.

That the wearable device 100 obtains the constant pressure control period A may include: The pressure sensor 213E detects that the pressure in the airbag 213G reaches 30 mmHg, to be specific, the pressure in the airbag 213G reaches the first constant pressure control point 30 mmHg. The duty cycle of the drive circuit 213B still remains at the first duty cycle. To keep the pressure in the airbag 213G at 30 mmHg, the drive circuit 213B may control the air release rate of the air valve 213D to be (v1-v2). In this case, the air inflation rate of the airbag 213G is v1, and the air release rate of the airbag 213G is (v1-v2)+v2=v1. To be specific, the air inflation rate of the airbag 213G is the same as the air release rate of the airbag 213G. The pressure in the airbag 213G remains unchanged and is fixed at 30 mmHg.

At 11s to 22s, the pressure in the airbag 213G remains at 30 mmHg. In this case, the average pressure increase rate of the pressure in the airbag 213G is 0.

Optionally, retention time within the linear pressure increase period A may be set to 12s.

It should be noted that, during the linear pressure increase period B, the pressure sensor 213E also obtains a peripheral arterial pulse wave signal of the user in real time.

That the wearable device 100 obtains the linear pressure increase period B may include: After the pressure sensor 213E detects that the pressure in the airbag 213G has remained at 30 mmHg for 12s, the wearable device 100 may control the duty cycle of the drive circuit 213B to be the first duty cycle, and control the air valve 213D to be closed. In response to the duty cycle of the drive circuit 213B being the first duty cycle, the drive circuit 213B may control the micropump 213C to inflate the airbag 213G at the air inflation rate v1. In addition, the micropump 213C slightly releases air. For example, the air release rate of the airbag 213G is v2. In this case, it can be learned that the pressure increase rate of the pressure in the airbag 213G is (v1-v2).

For example, as shown inFIG. 6, at 23s to 32s, the pressure in the airbag 213G uniformly increases to 60 mmHg. In this case, the average pressure increase rate of the pressure in the airbag 213G is 3 mmHg/s. The average pressure increase rate within the linear pressure increase period B is the same as the average pressure increase rate within the linear pressure increase period A.

It should be noted that, during the linear pressure increase period B, the pressure sensor 213E also obtains a peripheral arterial pulse wave signal of the user in real time.

That the wearable device 100 obtains the constant pressure control period B may include: The pressure sensor 213E detects that the pressure in the airbag 213G reaches 60 mmHg, to be specific, the pressure in the airbag 213G reaches the second constant pressure control point 60 mmHg. The duty cycle of the drive circuit 213B still remains at the first duty cycle. To keep the pressure in the airbag 213G at 60 mmHg, the drive circuit 213B may control the air release rate of the air valve 213D to be (v1-v2). In this case, the air inflation rate of the airbag 213G is v1, and the air release rate of the airbag 213G is (v1-v2)+v2=v1. To be specific, the air inflation rate of the airbag 213G is the same as the air release rate of the airbag 213G. The pressure in the airbag 213G remains unchanged and is fixed at 60 mmHg.

For example, as shown inFIG. 6, at 33s to 44s, the pressure in the airbag 213G remains at 60 mmHg. In this case, the average pressure increase rate of the pressure in the airbag 213G is 0.

Optionally, retention time within the constant pressure control period B may be set to 12s.

It should be noted that, during the constant pressure control period B, the pressure sensor 213E also obtains a peripheral arterial pulse wave signal of the user in real time. Within the constant pressure control period B, the pressure in the airbag 213G is fixed. Therefore, the peripheral arterial pulse wave signal obtained by the pressure sensor 213E has fewer lost frames than the peripheral arterial pulse wave signal obtained by the pressure sensor 213E within the linear pressure increase period B, and has good effect.

That the wearable device 100 obtains the linear pressure increase period C may include: After the pressure sensor 213E detects that the pressure in the airbag 213G has remained at 90 mmHg for 12s, the wearable device 100 may control the duty cycle of the drive circuit 213B to be the first duty cycle, and control the air valve 213D to be closed. In response to the duty cycle of the drive circuit 213B being the first duty cycle, the drive circuit 213B may control the micropump 213C to inflate the airbag 213G at the air inflation rate v1. In addition, the micropump 213C slightly releases air. For example, the air release rate of the airbag 213G is v2. In this case, it can be learned that the pressure increase rate of the pressure in the airbag 213G is (v1-v2).

For example, as shown inFIG. 6, at 45s to 54s, the pressure in the airbag 213G uniformly increases to 90 mmHg. In this case, the average pressure increase rate of the pressure in the airbag 213G is 3 mmHg/s. The average pressure increase rate within the linear pressure increase period C is the same as the average pressure increase rate within the linear pressure increase period B.

It should be noted that, during the linear pressure increase period C, the pressure sensor 213E also obtains a peripheral arterial pulse wave signal of the user in real time.

That the wearable device 100 obtains the constant pressure control period C may include: The pressure sensor 213E detects that the pressure in the airbag 213G reaches 90 mmHg, to be specific, the pressure in the airbag 213G reaches the third constant pressure control point 90 mmHg. The duty cycle of the drive circuit 213B still remains at the first duty cycle. To keep the pressure in the airbag 213G at 90 mmHg, the drive circuit 213B may control the air release rate of the air valve 213D to be (v1-v2). In this case, the air inflation rate of the airbag 213G is v1, and the air release rate of the airbag 213G is (v1-v2)+v2=v1. To be specific, the air inflation rate of the airbag 213G is the same as the air release rate of the airbag 213G. The pressure in the airbag 213G remains unchanged and is fixed at 90 mmHg.

For example, as shown in FIG. 6, at 55s to 66s, the pressure in the airbag 213G remains at 90 mmHg. In this case, the average pressure increase rate of the pressure in the airbag 213G is 0.

Optionally, retention time within the constant pressure control period C may be set to 12s.

It should be noted that, during the constant pressure control period C, the pressure sensor 213E also obtains a peripheral arterial pulse wave signal of the user in real time. Within the constant pressure control period C, the pressure in the airbag 213G is fixed. Therefore, the peripheral arterial pulse wave signal obtained by the pressure sensor 213E has fewer lost frames than the peripheral arterial pulse wave signal obtained by the pressure sensor 213E within the linear pressure increase period C, and has good effect.

That the wearable device 100 obtains the linear pressure increase period D may include: After the pressure sensor 213E detects that the pressure in the airbag 213G has remained at 90 mmHg for 12s, the wearable device 100 may control the duty cycle of the drive circuit 213B to be the first duty cycle, and control the air valve 213D to be closed. In response to the duty cycle of the drive circuit 213B being the first duty cycle, the drive circuit 213B may control the micropump 213C to inflate the airbag 213G at the air inflation rate v1. In addition, the micropump 213C slightly releases air. For example, the air release rate of the airbag 213G is v2. In this case, it can be learned that the pressure increase rate of the pressure in the airbag 213G is (v1-v2).

For example, as shown in FIG. 6 at 67s to 76s, the pressure in the airbag 213G uniformly increases to 120 mmHg. In this case, the average pressure increase rate of the pressure in the airbag 213G is 3 mmHg/s. The average pressure increase rate within the linear pressure increase period D is the same as the average pressure increase rate within the linear pressure increase period C.

It should be noted that, during the linear pressure increase period D, the pressure sensor 213E also obtains a peripheral arterial pulse wave signal of the user in real time.

That the wearable device 100 obtains the constant pressure control period D may include: The pressure sensor 213E detects that the pressure in the airbag 213G reaches 120 mmHg, to be specific, the pressure in the airbag 213G reaches the fourth constant pressure control point 120 mmHg. The duty cycle of the drive circuit 213B still remains at the first duty cycle. To keep the pressure in the airbag 213G at 120 mmHg, the drive circuit 213B may control the air release rate of the air valve 213D to be (v1-v2). In this case, the air inflation rate of the airbag 213G is v1, and the air release rate of the airbag 213G is (v1-v2)+v2=v1. To be specific, the air inflation rate of the airbag 213G is the same as the air release rate of the airbag 213G. The pressure in the airbag 213G remains unchanged and is fixed at 120 mmHg.

For example, as shown inFIG. 6, at 77s to 88s, the pressure in the airbag 213G remains at 120 mmHg. In this case, the average pressure increase rate of the pressure in the airbag 213G is 0.

Optionally, retention time within the constant pressure control period D may be set to 12s.

It should be noted that, during the constant pressure control period D, the pressure sensor 213E also obtains a peripheral arterial pulse wave signal of the user in real time. Within the constant pressure control period D, the pressure in the airbag 213G is fixed. Therefore, the peripheral arterial pulse wave signal obtained by the pressure sensor 213E has fewer lost frames than the peripheral arterial pulse wave signal obtained by the pressure sensor 213E within the linear pressure increase period D, and has good effect.

That the wearable device 100 obtains the linear pressure increase period E may include: After the pressure sensor 213E detects that the pressure in the airbag 213G remains at 120 mmHg for 12s, the wearable device 100 may control the duty cycle of the drive circuit 213B to be the first duty cycle, and control the air valve 213D to be closed. In response to the duty cycle of the drive circuit 213B being the first duty cycle, the drive circuit 213B may control the micropump 213C to inflate the airbag 213G at the air inflation rate v1. In addition, the micropump 213C slightly releases air. For example, the air release rate of the airbag 213G is v2. In this case, it can be learned that the pressure increase rate of the pressure in the airbag 213G is (v1-v2).

For example, as shown inFIG. 6, at 89s and later, the pressure in the airbag 213G uniformly increases to a maximum value, and the average pressure increase rate of the pressure in the airbag 213G is 3 mmHg/s. The average pressure increase rate within the linear pressure increase period E is the same as the average pressure increase rate within the linear pressure increase period D.

It should be noted that, during the linear pressure increase period E, the pressure sensor 213E also obtains a peripheral arterial pulse wave signal of the user in real time.

The foregoing steps are repeated until the micropump 213C stops inflating the airbag 213G, and blood pressure measurement ends.

The foregoing steps are repeated until the micropump 213C stops inflating the airbag 213G, and blood pressure measurement ends.

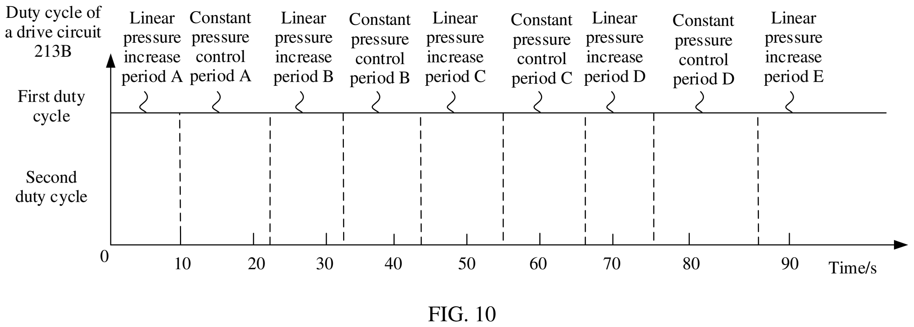

FIG. 10 is a diagram of duty cycles corresponding to the drive circuit 213B within linear pressure increase periods and constant pressure control periods. All of duty cycles of the drive circuit 213B within the linear pressure increase period A, the linear pressure increase period B, the linear pressure increase period C, the linear pressure increase period D, the linear pressure increase period E, the constant pressure control period A, the constant pressure control period B, the constant pressure control period C, and the constant pressure control period D are the first duty cycle.

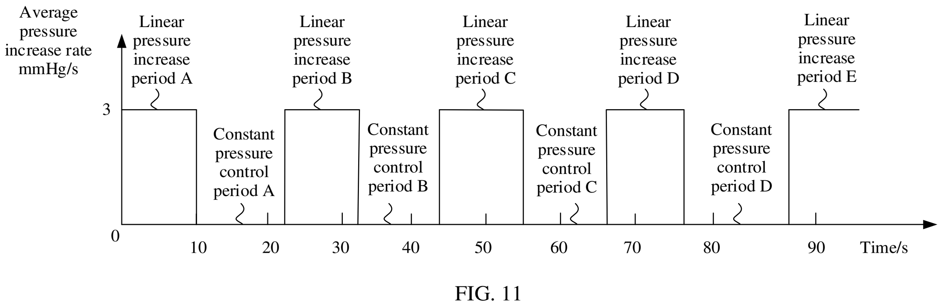

FIG. 11 shows average pressure increase rates of the airbag 213G within linear pressure increase periods and constant pressure control periods. All of average pressure increase rates of the airbag 213G within the linear pressure increase period A, the linear pressure increase period B, the linear pressure increase period C, the linear pressure increase period D, and the linear pressure increase period E are 3 mmHg/s. All of average pressure increase rates of the airbag 213G within the constant pressure control period A, the constant pressure control period B, the constant pressure control period C, and the constant pressure control period D are 0. To be specific, in the method 2, average pressure increase rates of the airbag 213G within linear pressure increase periods are the same, average pressure increase rates of the airbag 213G within constant pressure control periods are close to 0, and the average pressure increase rates of the airbag 213G within the constant pressure control periods are fixed.

It should be noted that, in addition to the five linear pressure increase periods and the four constant pressure control periods, more or fewer linear pressure increase periods and constant pressure control periods may alternatively be set for the wearable device 100. Details are not described herein in this application.

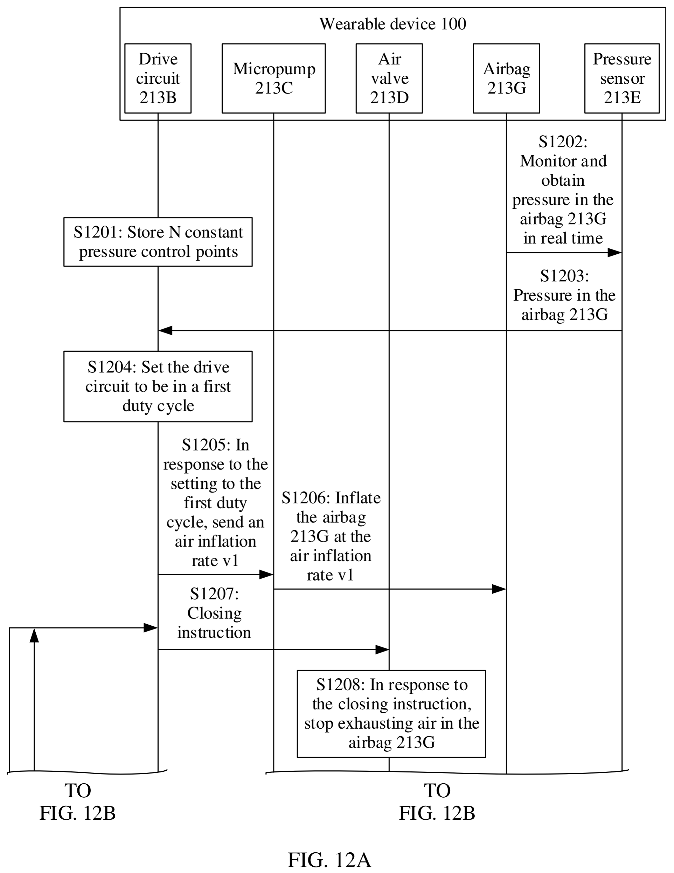

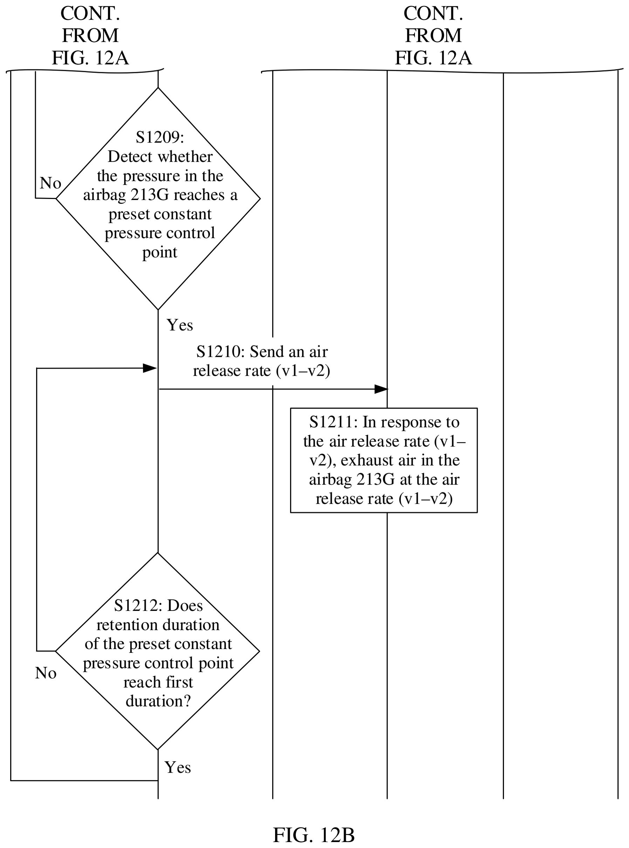

**FIG. 12A** **and** **FIG. 12B** **are a schematic flowchart of another method for obtaining N linear pressure increase periods and N constant pressure control periods by a wearable device 100.**

As shown in FIG. 12A and FIG. 12B, the wearable device 100 includes a drive circuit 213B, a micropump 213C, an air valve 213D, an airbag 213G, and a pressure sensor 213E.

Within a linear pressure increase period, the air valve 213D is in a closed state. Within a constant pressure control period, the air valve 213D is in an open state.

For descriptions of S1201 to S1203, refer to the descriptions of S901 to S903. Details are not described herein again in this application.

S1204: The drive circuit 213B sets the drive circuit to be in a first duty cycle.

S1205: In response to the setting to the first duty cycle, the drive circuit 213B sends an air inflation rate v1 to the micropump 213C.

S1206: The micropump 213C inflates the airbag 213G at the air inflation rate v1.

The drive circuit 213B receives an instruction and starts to measure blood pressure. When starting to measure blood pressure, the drive circuit 213B sets the drive circuit to be in the first duty cycle. In response to the setting to the first duty cycle, the drive circuit 213B sends the air inflation rate v1 to the micropump 213C, so that the micropump 213C inflates the airbag 213G at the air inflation rate v1. In addition, the micropump 213C slightly releases air. For example, an air release rate of the airbag 213G is v2. In this case, it can be learned that a pressure increase rate of the pressure in the airbag 213G is (v1-v2), so that the pressure in the airbag 213G uniformly increases at the pressure increase rate (v1-v2).

It should be noted that, in the method 2, during blood pressure measurement, the micropump 213C continuously inflates the airbag 213G at the air inflation rate v1.

S1207: The drive circuit 213B sends a closing instruction to the air valve 213D.

S1208: In response to the closing instruction, the air valve 213D stops exhausting air in the airbag 213G.

Optionally, S1207 and S1208 may alternatively be performed before S1204 to S1206, or S1207 and S1208 may be performed simultaneously with S1204 to S1206. This is not limited in this application.

The drive circuit 213B receives an instruction and starts to measure blood pressure. When starting to measure blood pressure, the drive circuit 213B sends the closing instruction to the air valve 213D, and in response to the closing instruction, the air valve 213D stops exhausting air in the airbag 213G.

To be specific, when the pressure in the airbag 213G does not reach a preset constant pressure control point, the air valve 213D is in the closed state.

S1209: The drive circuit 213B needs to detect whether the pressure in the airbag 213G reaches the preset constant pressure control point.

The preset constant pressure control point may be any one of the N constant pressure control points. For example, the preset constant pressure control point may be 30 mmHg, 60 mmHg, 90 mmHg, or 120 mmHg.

The pressure sensor 213E may periodically or aperiodically send the pressure in the airbag 213G to the drive circuit 213B, and the drive circuit 213B needs to detect whether the pressure in the airbag 213G reaches the preset constant pressure control point.

In this way, when the pressure in the airbag 213G reaches the preset constant pressure control point, the drive circuit 213B may adjust an air release rate of the air valve 213D to keep the pressure in the airbag 213G constant, to obtain a peripheral arterial pulse wave signal within a constant pressure control period.

When the pressure in the airbag 213G does not reach the preset constant pressure control point, the drive circuit 213B may not open the air valve 213D, so that the pressure in the airbag 213G uniformly increases at the pressure increase rate (v1-v2).

When the pressure in the airbag 213G reaches the preset constant pressure control point, S1210 is performed.

When the pressure in the airbag 213G does not reach the preset constant pressure control point, S1207 is performed. Alternatively, when the pressure in the airbag 213G does not reach the preset constant pressure control point, S1207 is not performed, and the air valve 213D is still in the closed state.

S1210: The drive circuit 213B sends an air release rate (v1-v2) to the air valve 213D.

S1211: In response to the air release rate (v1-v2), the air valve 213D exhausts air in the airbag 213G at the air release rate (v1-v2).

When the pressure in the airbag 213G reaches the preset constant pressure control point, the drive circuit 213B sends the air release rate (v1-v2) to the air valve 213D. In response to the air release rate (v1-v2), the air valve 213D exhausts air in the airbag 213G at the air release rate (v1-v2).

In addition, the micropump 213C slightly releases air. For example, the air release rate of the airbag 213G is v2. In this case, it can be learned that the air release rate of the airbag 213G is v1 and is the same as the air inflation rate of the airbag 213G. To be specific, the pressure increase rate of the airbag 213G is 0, and the pressure in the airbag 213G remains unchanged.

In this way, when the pressure in the airbag 213G reaches the preset constant pressure control point, the air valve 213D may be open to exhaust air in the airbag 213G, so that the air inflation rate of the airbag 213G is equal to the air release rate of the airbag 213G. The pressure sensor 213E may obtain a peripheral arterial pulse wave signal within a constant pressure control period.

S1212: The drive circuit 213B needs to determine whether retention duration of the preset constant pressure control point reaches first duration.

After the pressure in the airbag 213G reaches the preset constant pressure control point, the micropump 213C needs to determine whether the retention duration of the preset constant pressure control point reaches the first duration.

When the retention duration of the preset constant pressure control point reaches the first duration, S1207 is performed. To be specific, the drive circuit 213B sends a closing instruction to the air valve 213D again, so that the air valve 213D stops exhausting air in the airbag 213G. In some embodiments, after blood pressure measurement is completed and the micropump 213C stops inflating the airbag 213G, the wearable device 100 may open the air valve 213D to increase the air release rate of the airbag 213G.

When the retention duration of the preset constant pressure control point does not reach the first duration, S1210 is performed. Alternatively, when the retention duration of the preset constant pressure control point does not reach the first duration, S1210 is not performed. To be specific, the air valve 213D is still closed, so that the pressure in the airbag 213G continues to remain constant.

In some embodiments, after blood pressure measurement is completed and the micropump 213C stops inflating the airbag 213G, the wearable device 100 may open the air valve 213D to increase the air release rate of the airbag 213G.

2. The wearable device 100 obtains a peripheral arterial blood pressure value **based on airbag pressure signals corresponding to the N linear pressure increase periods and peripheral arterial pulse wave signals corresponding to the N linear pressure increase periods.**

It can be learned from the foregoing descriptions that, in a process in which the wearable device 100 measures blood pressure by inflating the airbag 213G through the micropump 213C, the pressure sensor 213E also continuously collects a peripheral arterial pulse wave signal.

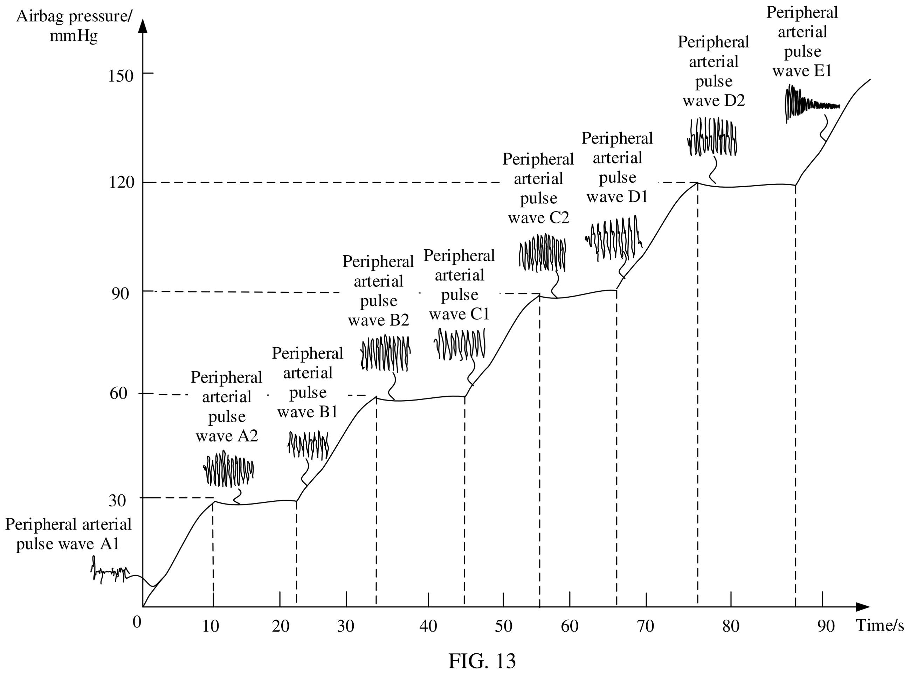

As shown in FIG. 13, the pressure sensor 213E collects a peripheral arterial pulse wave A1 within the linear pressure increase period A, and the pressure sensor 213E collects a peripheral arterial pulse wave A2 within the constant pressure control period A.

The pressure sensor 213E collects a peripheral arterial pulse wave B1 within the linear pressure increase period B, and the pressure sensor 213E collects a peripheral arterial pulse wave B2 within the constant pressure control period B.

The pressure sensor 213E collects a peripheral arterial pulse wave C1 within the linear pressure increase period C, and the pressure sensor 213E collects a peripheral arterial pulse wave C2 within the constant pressure control period C.

The pressure sensor 213E collects a peripheral arterial pulse wave D1 within the linear pressure increase period D, and the pressure sensor 213E collects a peripheral arterial pulse wave D2 within the constant pressure control period D.

The pressure sensor 213E collects a peripheral arterial pulse wave E1 within the linear pressure increase period E.

The wearable device 100 may obtain the peripheral arterial pulse wave signals corresponding to the N linear pressure increase periods, and obtain the peripheral arterial blood pressure value based on the peripheral arterial pulse wave signals corresponding to the N linear pressure increase periods.

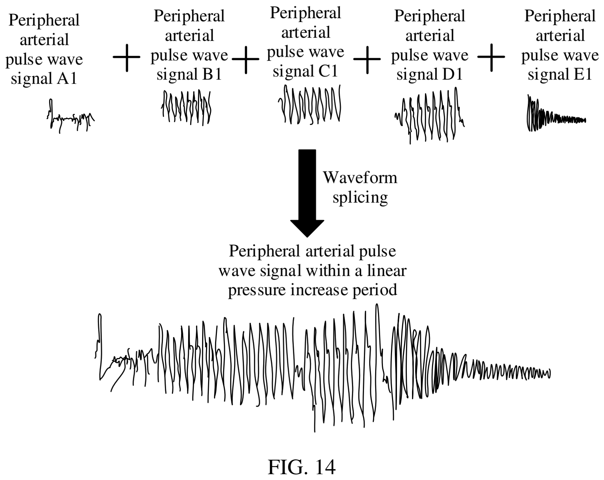

Specifically, as shown in FIG. 14, the wearable device 100 may obtain the peripheral arterial pulse wave signals corresponding to the N linear pressure increase periods, for example, the peripheral arterial pulse wave signal A1, the peripheral arterial pulse wave signal B1, the peripheral arterial pulse wave signal C1, the peripheral arterial pulse wave signal D1, and the peripheral arterial pulse wave signal E1. Then the wearable device 100 splices, in chronological order, the peripheral arterial pulse wave signals corresponding to the N linear pressure increase periods into a complete peripheral arterial pulse wave signal. The wearable device 100 may obtain the peripheral arterial blood pressure value based on the peripheral arterial pulse wave signals within the linear pressure increase periods and blood pressure change signals within the linear pressure increase periods.

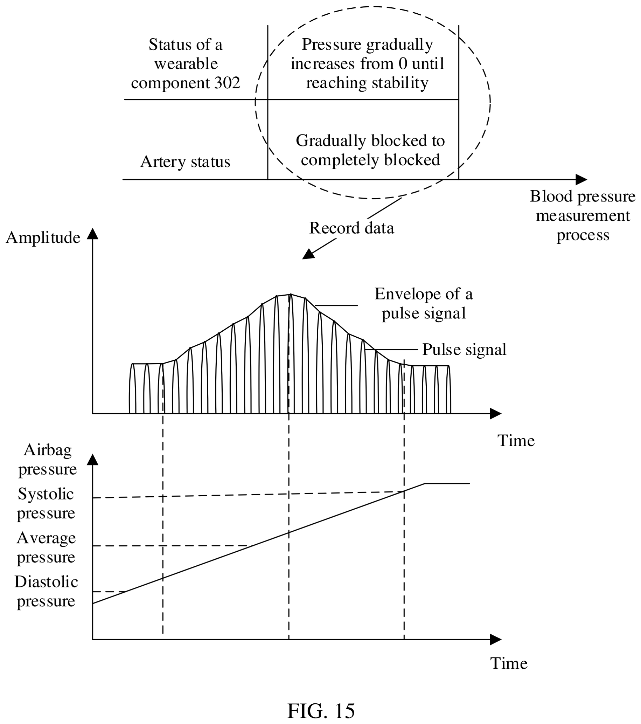

FIG. 15 is a diagram of obtaining a peripheral arterial blood pressure value according to an embodiment of this application.

As shown in FIG. 15, in a process in which the wearable device 100 inflates the wearable component 202 to temporarily block an upper-limb artery, the wearable component 202 is in a state in which pressure gradually increases until reaching stability, and the artery is in a state of being gradually blocked to being completely blocked. When the wearable component 202 is in the state in which the pressure gradually increases until reaching stability, a pressure value and a pulse signal of the wearable component 202 are recorded. When the pressure value of the wearable component 202 gradually increases and diastolic pressure is less than average pressure, the pulse signal is a fine oscillation wave. When the pressure value of the wearable component 202 continues to gradually increase and is greater than the diastolic pressure and less than the average pressure, an amplitude of the pulse signal gradually increases. When the pressure value of the wearable component 202 is equal to the average pressure, the amplitude of the pulse signal reaches a maximum value. When the pressure value of the wearable component 202 gradually increases and is greater than the average pressure and less than systolic pressure, the artery is gradually blocked, and the amplitude of the pulse signal continuously decreases. When the pressure value of the wearable component 202 is greater than or equal to the systolic pressure, the artery is blocked, and the pulse signal is a fine oscillation wave. Therefore, the wearable device 100 may determine the systolic pressure and the diastolic pressure of the user based on the pressure value of the wearable component 202 and a change in the amplitude of the pulse signal. In a possible implementation, the pressure value and the pulse signal of the wearable component 202 may be determined by a built-in pressure sensor in the wearable device 100.

**3. The wearable device 100 obtains a central arterial pulse wave signal based on peripheral arterial pulse wave signals corresponding to the N constant pressure control periods.**

It can be learned from the descriptions in the embodiment of FIG. 14 that, during blood pressure measurement, the wearable device 100 may obtain the peripheral arterial pulse wave signals corresponding to the N linear pressure increase periods. Within a constant pressure control period, pressure in the airbag 213G remains constant, and a peripheral arterial pulse wave signal obtained by the pressure sensor 213E has a few lost frames and has good quality. The wearable device 100 may obtain a central arterial pulse wave signal based on the peripheral arterial pulse wave signal obtained within the constant pressure control period, to analyze whether the user has a cardiovascular risk.

It can be learned from the embodiment of FIG. 13 that the wearable device 100 may obtain the peripheral arterial pulse wave signal A2 corresponding to the constant pressure control period A, the peripheral arterial pulse wave signal B2 corresponding to the constant pressure control period B, the peripheral arterial pulse wave signal C2 corresponding to the constant pressure control period C, and the peripheral arterial pulse wave signal D2 corresponding to the constant pressure control period D.

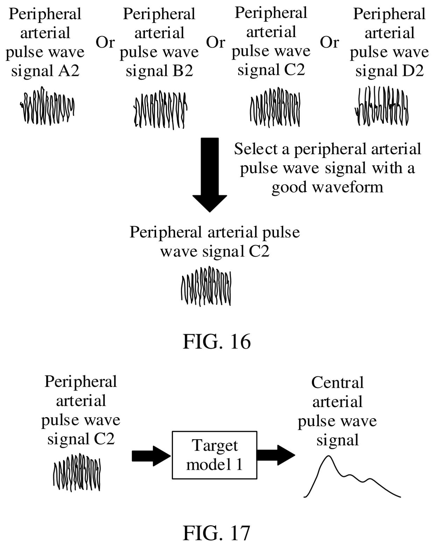

In a possible implementation, as shown in FIG. 16, the wearable device 100 may select, from the peripheral arterial pulse wave signals corresponding to the foregoing constant pressure control periods, a peripheral arterial pulse wave signal with a good waveform. A selection rule may be: An amplitude of the peripheral arterial pulse wave signal changes slightly. The selection rule may alternatively be another rule. This is not limited in this application.

For example, as shown in FIG. 16, the wearable device 100 may select the peripheral arterial pulse wave signal C2 with a good waveform from the peripheral arterial pulse wave signal A2, the peripheral arterial pulse wave signal B2, the peripheral arterial pulse wave signal C2, and the peripheral arterial pulse wave signal D2.

Then the wearable device 100 may obtain a central arterial pulse wave signal based on the peripheral arterial pulse wave signal C2.

In some embodiments, the wearable device 100 may obtain the central arterial pulse wave signal based on the peripheral arterial pulse wave signal C2 through a target model 1.

Optionally, the target model 1 may include a transfer function.

The target model 1 may be obtained through training by the wearable device 100. Alternatively, the target model 1 may be obtained through training by a server or another device, and the wearable device 100 may directly obtain the trained target model 1 and use the target model 1.

The following describes how to train the target model 1.

First, a large amount of training data is obtained. The training data includes a peripheral arterial pulse wave signal and a central arterial pulse wave signal corresponding to the peripheral arterial pulse wave signal. The peripheral arterial pulse wave signal in the training data is input to the target model 1, and the target model 1 may output a peripheral arterial pulse wave signal. A similarity between the peripheral arterial pulse wave signal output by the target model 1 and the peripheral arterial pulse wave signal in the training data is obtained through comparison. When the similarity is less than a preset value, parameter information of the target model 1 is adjusted. The peripheral arterial pulse wave signal in the training data is input to the target model 1 again, and the target model 1 may output a peripheral arterial pulse wave signal. A similarity between the peripheral arterial pulse wave signal output by the target model 1 and the peripheral arterial pulse wave signal in the training data is obtained through comparison. When the similarity is less than the preset value, the foregoing steps are repeated until a similarity between a peripheral arterial pulse wave signal output by the target model 1 and the peripheral arterial pulse wave signal in the training data is greater than the preset value, and training of the target model 1 is completed. An input for the trained target model 1 is a peripheral arterial pulse wave signal, and an output of the trained target model 1 is a central arterial pulse wave signal.

The wearable device 100 may obtain the central arterial pulse wave signal based on the trained target model 1 and the peripheral arterial pulse wave signal C2.

For example, as shown in FIG. 17, after the wearable device 100 obtains the peripheral arterial pulse wave signal C2, the wearable device 100 may input the peripheral arterial pulse wave signal C2 to the trained target model 1, and the trained target model 1 may output the central arterial pulse wave signal corresponding to the peripheral arterial pulse wave signal C2.

Optionally, after the wearable device 100 obtains the trained target model 1, the wearable device 100 may periodically or aperiodically update the target model 1, to improve accuracy of obtaining, by the target model 1, a central arterial pulse wave signal based on a peripheral arterial pulse wave signal.

In some embodiments, after the wearable device 100 obtains the central arterial pulse wave signal, the wearable device 100 may assess a physical status of the user based on the central arterial pulse wave signal. For example, the wearable device 100 may obtain, based on the central arterial pulse wave signal, parameter information for assessing a cardiovascular disease. The parameter information for assessing the cardiovascular disease includes but is not limited to one or more of the following: a heart rate, vascular wall elasticity, a cardiac ejection capability, peripheral vascular resistance, and the like.

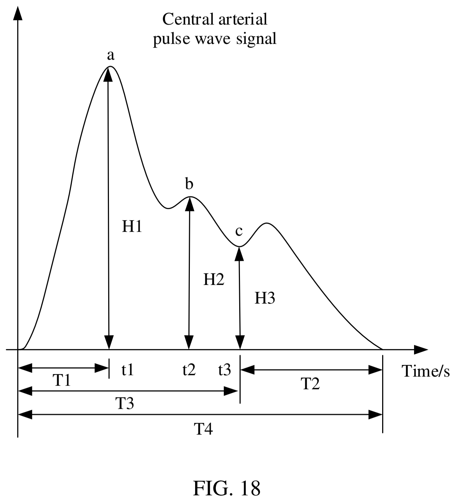

FIG. 18 is a diagram of a central arterial pulse wave signal obtained within one cycle.

The wearable device 100 may determine, based on time corresponding to a feature point of the central arterial pulse wave signal and a central arterial pressure value, parameter information for assessing a cardiovascular disease.

For example, as shown in FIG. 18, the wearable device 100 may obtain a feature point a, a feature point b, and a feature point c from the central arterial pulse wave signal. The feature point a represents a position of maximum central arterial pressure at time t1, and a maximum value of central arterial pressure is H1. The feature point b represents a position of a second largest value of the central arterial pressure at time t2, and the second largest value of the central arterial pressure is H2. The feature point c represents a position of minimum central arterial pressure at time t3, and a minimum value of the central arterial pressure is H3.

H2/H1 may indicate the vascular wall elasticity, H3/H1 may indicate the peripheral vascular resistance, T1/T4 may indicate the cardiac ejection capability, T3/T2 may indicate the heart rate, and so on.

In addition to the parameter information such as the heart rate, the vascular wall elasticity, the cardiac ejection capability, and the peripheral vascular resistance, the wearable device 100 may alternatively obtain, from the central arterial pulse wave signal, more or less other parameter information for assessing the cardiovascular disease. This is not limited in this application either.

**4. The wearable device 100 obtains a central arterial blood pressure value based on the peripheral arterial blood pressure value and the central arterial pulse wave signal.**

The peripheral arterial blood pressure value may include peripheral arterial systolic pressure, peripheral arterial diastolic pressure, and peripheral arterial average pressure.

The central arterial blood pressure value may include central arterial systolic pressure, central arterial diastolic pressure, and central arterial average pressure.

The wearable device 100 may obtain the central arterial blood pressure value based on the peripheral arterial blood pressure value and the central arterial pulse wave signal.

In some embodiments, the wearable device 100 may obtain the central arterial blood pressure value based on the peripheral arterial blood pressure value and the central arterial pulse wave signal through a target model 2.

Optionally, the target model 2 may include a transfer function.

The target model 2 may be obtained through training by the wearable device 100. Alternatively, the target model 2 may be obtained through training by a server or another device, and the wearable device 100 may directly obtain the trained target model 2 and use the target model 2.

The following describes how to train the target model 2.

First, a large amount of training data is obtained. The training data includes a peripheral arterial blood pressure value, a central arterial pulse wave signal, and a central arterial blood pressure value corresponding to the peripheral arterial blood pressure value. The peripheral arterial blood pressure value and the central arterial pulse wave signal in the training data are input to the target model 2, and the target model 2 may output a central arterial blood pressure value. A difference between the central arterial blood pressure value output by the target model 2 and the central arterial blood pressure value corresponding to the peripheral arterial blood pressure value in the training data is obtained through comparison. When the difference is greater than a preset value, parameter information of the target model 2 is adjusted. The peripheral arterial blood pressure value and the central arterial pulse wave signal in the training data are input to the target model 2 again, and the target model 2 may output a central arterial blood pressure value. A similarity between the central arterial blood pressure value output by the target model 1 and the central arterial blood pressure value corresponding to the peripheral arterial blood pressure value in the training data is obtained through comparison. When the difference is greater than the preset value, the foregoing steps are repeated until a difference between a central arterial blood pressure value output by the target model 2 and the central arterial blood pressure value corresponding to the peripheral arterial blood pressure value in the training data is less than the preset value, and training of the target model 2 is completed. An input for the trained target model 2 is a peripheral arterial blood pressure value and a central arterial pulse wave signal, and an output of the trained target model 2 is a central arterial blood pressure value.

The wearable device 100 may obtain the central arterial pulse wave signal based on the trained target model 2, the peripheral arterial blood pressure value, and the central arterial pulse wave signal.

In some embodiments, in addition to the foregoing two methods for obtaining a peripheral arterial pulse wave signal within a constant pressure control period to obtain a central arterial pulse wave signal,
in another embodiment, the wearable device 100 may alternatively obtain a peripheral arterial pulse wave signal within a constant pressure control period based on a PPG module, to obtain a central arterial pulse wave signal.

Embodiments of this application provide two other methods for obtaining a peripheral arterial pulse wave signal within a constant pressure control period based on the PPG module.

**Method 3: When pressure in the airbag 213G remains constant, the peripheral arterial pulse wave signal within the constant pressure control period is obtained based on the PPG module.**

For example, the pressure in the airbag 213G may be constant at approximately average pressure. In addition to the average pressure, the pressure in the airbag 213G may alternatively be constant at another pressure value. This is not limited in this application.

When starting to measure blood pressure, the wearable device 100 may inflate the airbag 213G at an air inflation rate v1 through the micropump 213C. When detecting that the pressure in the airbag 213G is equal to the average pressure, the micropump 213C inflates the airbag 213G at an air inflation rate v1-v2; or the micropump 213C continues to inflate the airbag 213G at the air inflation rate v1, but the wearable device 100 needs to open the air valve 213D and control the air valve 213D to exhaust air in the airbag 213G at an air release rate v1-v2, so that the pressure in the airbag 213G is stable at approximately the average pressure.

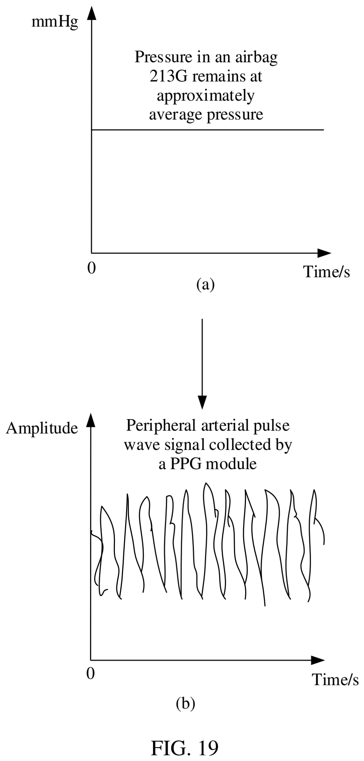

As shown in (a) and (b) in FIG. 19, after the pressure in the airbag 213G is stable at approximately the average pressure, the wearable device 100 may collect a peripheral arterial pulse wave signal through the PPG module. The peripheral arterial pulse wave signal collected by the PPG module is collected when the pressure in the airbag 213G is constant.

In some embodiments, after obtaining the peripheral arterial pulse wave signal, the wearable device 100 may obtain a central arterial pulse wave signal based on the peripheral arterial pulse wave signal shown in (b) in FIG. 19, to analyze whether the user has a cardiovascular risk. For how the wearable device 100 obtains the central arterial pulse wave signal based on the peripheral arterial pulse wave signal shown in (b) in 19 and how the wearable device 100 obtains, based on the central arterial pulse wave signal, parameter information for assessing a cardiovascular disease, refer to the descriptions of FIG. 17 and FIG. 18. Details are not described herein again in this application.

After obtaining the peripheral arterial pulse wave signal, the wearable device 100 may determine a peripheral arterial pressure signal based on the peripheral arterial pulse wave signal, and then obtain a peripheral arterial pressure value based on the peripheral arterial pressure signal.

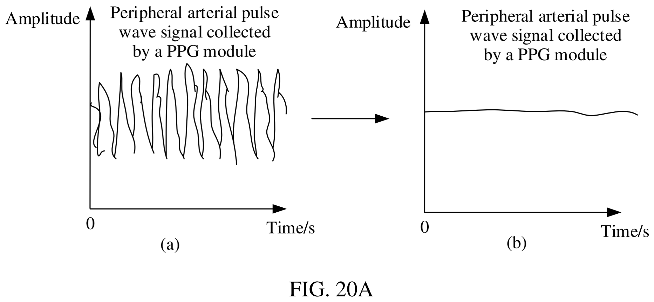

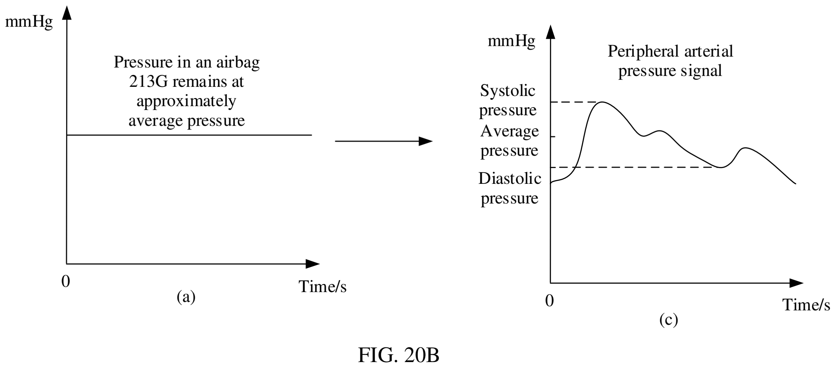

Specifically, as shown in (a) and (b) in FIG. 20A, when the peripheral arterial pulse wave signal collected by the PPG module changes from a waveform shown in (a) in FIG. 20A to a waveform shown in (b) in FIG. 20A, a blood pressure change trend may be obtained, so that the peripheral arterial pressure signal changes from a waveform shown in (a) in FIG. 20B to a waveform shown in (b) in FIG. 20B.

The waveform shown in (b) in FIG. 20B is a peripheral arterial pressure signal within one cycle, and the wearable device 100 may obtain the peripheral arterial pressure value based on the peripheral arterial pressure signal shown in (b) in FIG. 20B. The peripheral arterial pressure value includes peripheral arterial systolic pressure and peripheral arterial diastolic pressure.

As shown in (b) in FIG. 20B, a maximum peak value of the peripheral arterial pressure signal is systolic pressure, and a minimum trough value of the peripheral arterial pressure signal is diastolic pressure.

In some embodiments, after obtaining the peripheral arterial pressure value and the central arterial pulse wave signal, the wearable device 100 may obtain a central arterial pressure value based on the peripheral arterial pressure value and the central arterial pulse wave signal. For details, refer to the foregoing descriptions. Details are not described herein again in this application.

**Method 4: The wearable device 100 obtains a central arterial pulse wave signal, a peripheral arterial blood pressure value, and a central arterial blood pressure value based on a PPG signal collected by the PPG module.**

In the method 1 to the method 3, the wearable device 100 measures blood pressure of the user by inflating and deflating the airbag, affecting rest of the user. To avoid impact on the user, the wearable device 100 may obtain the blood pressure of the user by measuring a PPG signal, to reduce impact on sleep of the user.

In some embodiments, the wearable device 100 may obtain, through a target model 3, a central arterial pulse wave signal, a peripheral arterial blood pressure value, and a central arterial blood pressure value based on a PPG signal collected by the PPG module.

The target model 3 represents a correspondence between a PPG signal and a central arterial pulse wave signal, a peripheral arterial blood pressure value, and a central arterial blood pressure value.

For example, the target model 3 is preconfigured on the wearable device 100 before the wearable device 100 is delivered from a factory, or the wearable device 100 may dynamically obtain the target model 3 from a server. An input for the target model 3 may be a PPG signal measured by the wearable device 100 through the PPG module, and an output of the target model 3 may be a central arterial pulse wave signal, a peripheral arterial blood pressure value, and a central arterial blood pressure value. In other words, when a PPG signal measured by the wearable device 100 is input to the target model 3, a central arterial pulse wave signal, a peripheral arterial blood pressure value, and a central arterial blood pressure value may be obtained through the target model 3.

The following describes a training process of the target model 3.

The training process of the target model 3 specifically includes: A large amount of training data is obtained. The training data includes a PPG signal, and a central arterial pulse wave signal, a peripheral arterial blood pressure value, and a central arterial blood pressure value that correspond to the PPG signal. The PPG signal in the training data is input to the target model 3, and the target model 3 may output a central arterial pulse wave signal, a peripheral arterial blood pressure value, and a central arterial blood pressure value. A similarity between the central arterial pulse wave signal, the peripheral arterial blood pressure value, and the central arterial blood pressure value that are output by the target model 1 and the central arterial pulse wave signal, the peripheral arterial blood pressure value, and the central arterial blood pressure value that are in the training data is obtained through comparison. When the similarity is less than a preset value, parameter information of the target model 3 is adjusted. The PPG signal in the training data is input to the target model 3 again, and the target model 3 may output a central arterial pulse wave signal, a peripheral arterial blood pressure value, and a central arterial blood pressure value. A similarity between the central arterial pulse wave signal, the peripheral arterial blood pressure value, and the central arterial blood pressure value that are output by the target model 3 and the central arterial pulse wave signal, the peripheral arterial blood pressure value, and the central arterial blood pressure value that are in the training data is obtained through comparison. When the similarity is less than the preset value, the foregoing steps are repeated until a similarity between a central arterial pulse wave signal, a peripheral arterial blood pressure value, and a central arterial blood pressure value that are output by the target model 3 and the central arterial pulse wave signal, the peripheral arterial blood pressure value, and the central arterial blood pressure value that are in the training data is greater than the preset value, and training of the target model 3 is completed. An input for the trained target model 3 is a PPG signal, and an output of the trained target model 3 is a central arterial pulse wave signal, a peripheral arterial blood pressure value, and a central arterial blood pressure value.

In some embodiments, to improve accuracy of the target model 3, the target model 3 may be periodically or aperiodically updated by using a plurality of PPG signals and central arterial pulse wave signals, peripheral arterial blood pressure values, and central arterial blood pressure values that correspond to the PPG signals, to obtain a target model 4. The target model 4 is obtained by updating the target model 3 based on the plurality of PPG signals of the user and the central arterial pulse wave signals, the peripheral arterial blood pressure values, and the central arterial blood pressure values that correspond to the PPG signals. Therefore, a central arterial pulse wave signal, a peripheral arterial blood pressure value, and a central arterial blood pressure value of the user that are obtained based on a PPG signal through the target model 4 are more accurate than those obtained through the target model 3.

**In some embodiments, after obtaining a blood pressure measurement value and parameter information for assessing a cardiovascular disease, the wearable device 100 may display the blood pressure measurement value and the parameter information for assessing the cardiovascular disease.**

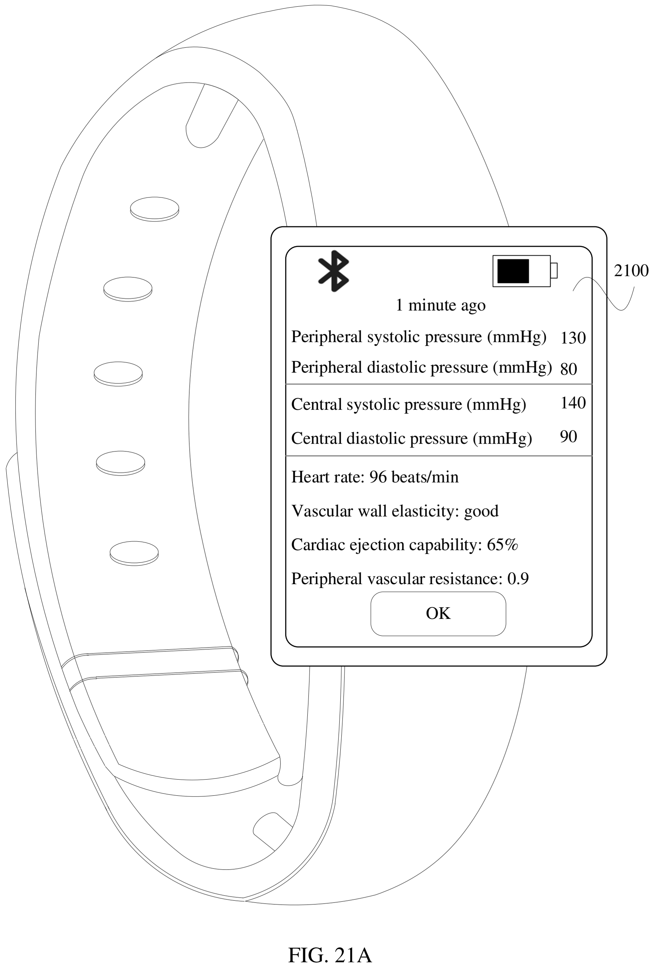

In some embodiments, after the wearable device 100 obtains a blood pressure measurement value and parameter information for assessing a cardiovascular disease, the wearable device 100 may display a user interface 2100 shown in FIG. 21A. The user interface 2100 includes a peripheral blood pressure measurement value, a central blood pressure measurement value, and the parameter information for assessing the cardiovascular disease. The blood pressure measurement value may include systolic pressure and diastolic pressure. For example, peripheral arterial systolic pressure may be 130 mmHg, and peripheral arterial diastolic pressure may be 80 mmHg. Central arterial systolic pressure may be 140 mmHg, and central arterial diastolic pressure may be 90 mmHg. The parameter information for assessing the cardiovascular disease may include: A heart rate is 96 beats/min, vascular wall elasticity is good, a cardiac ejection capability is 65%, and peripheral vascular resistance is 0.9.

In another embodiment, after the wearable device 100 obtains a blood pressure measurement value and parameter information for assessing a cardiovascular disease, the wearable device 100 may send the blood pressure measurement value and the parameter information for assessing the cardiovascular disease to an electronic device, and the electronic device displays the blood pressure measurement value and the parameter information for assessing the cardiovascular disease.

In some embodiments, the wearable device 100 may store a blood pressure measurement value and parameter information for assessing a cardiovascular disease that are within first duration before current time. A blood pressure measurement value and parameter information for assessing a cardiovascular disease that exceed the first duration are deleted, to save storage space of the wearable device 100.

Optionally, the wearable device 100 may also receive a user operation of viewing a blood pressure measurement value and parameter information for assessing a cardiovascular disease that are within a specific time period.

For example, the specific time period may be 24 hours.

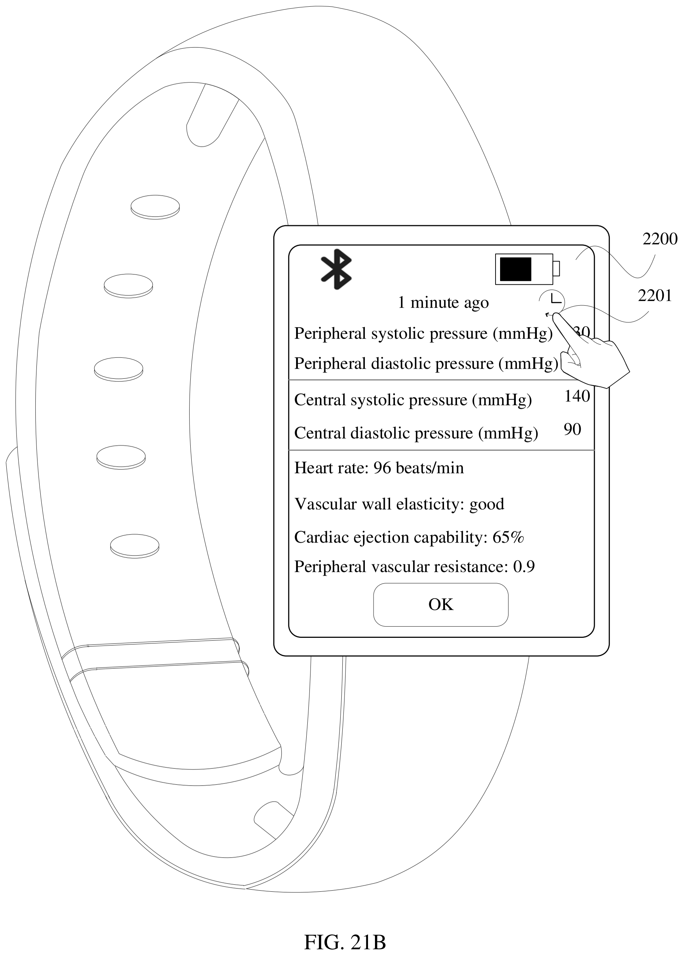

As shown in FIG. 21B, after the wearable device 100 obtains a blood pressure measurement value and parameter information for assessing a cardiovascular disease, the wearable device 100 may display a user interface 2200 shown in FIG. 21B. The user interface 2200 is similar to the user interface 2100. A difference lies in that the user interface 2200 further includes an icon 2201. The icon 2201 is used to display a blood pressure measurement value and parameter information for assessing a cardiovascular disease that are within a specific time period.

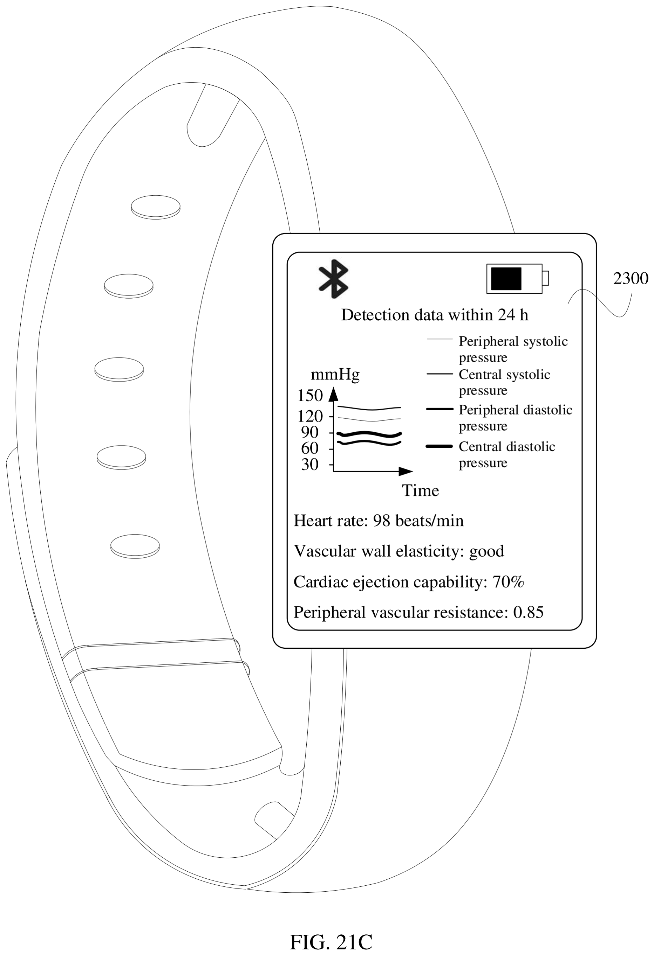

As shown in FIG. 21B, the wearable device 100 may receive an input operation (for example, tapping) of the user for the icon 2201 on the user interface 2200. In response to the input operation of the user, the wearable device 100 may obtain a blood pressure measurement value of the user and parameter information for assessing a cardiovascular disease that are within a specific time period, and display a user interface 2300 shown in FIG. 21C.

The user interface 2300 includes a graphical display area for blood pressure measurement values within 24 hours. The graphical display area includes a curve of peripheral arterial systolic pressure measurement values within 24 hours, a curve of central arterial systolic pressure measurement values within 24 hours, a curve of peripheral arterial diastolic pressure measurement values within 24 hours, and a curve of central arterial diastolic pressure measurement values within 24 hours. A blood pressure change trend of the user within 24 hours may be directly viewed in the graphical display area.

The user interface 2300 further includes parameter information for assessing a cardiovascular disease within 24 hours. For example, a heart rate is 98 beats/min, vascular wall elasticity is good, a cardiac ejection capability is 70%, and peripheral vascular resistance is 0.85.

In addition to 24 hours, the wearable device 100 may alternatively display a blood pressure monitoring value within another longer or shorter time period. This is not limited in this application.

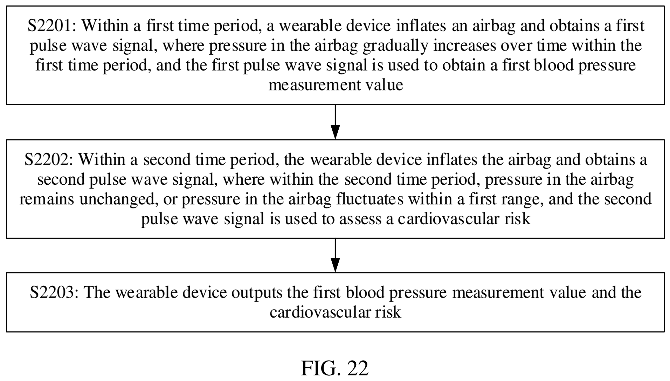

FIG. 22 is a schematic flowchart of a blood pressure measurement method according to this application.

S2201: Within a first time period, a wearable device inflates an airbag and obtains a first pulse wave signal, where pressure in the airbag gradually increases over time within the first time period, and the first pulse wave signal is used to obtain a first blood pressure measurement value.

The first blood pressure measurement value may be a blood pressure value, measured by the wearable device, of a part wearing the wearable device. For example, when the wearable device is worn on a wrist of a user, the first blood pressure measurement value may be a blood pressure value of the wrist of the user.

The first time period may be a time period corresponding to one or more linear pressure increase periods shown in FIG. 6.

The first pulse wave signal may be a peripheral arterial pulse wave corresponding to one or more linear pressure increase periods shown in FIG. 13.

S2202: Within a second time period, the wearable device inflates the airbag and obtains a second pulse wave signal, where within the second time period, pressure in the airbag remains unchanged, or pressure in the airbag fluctuates within a first range, and the second pulse wave signal is used to assess a cardiovascular risk.

The second time period may be a time period corresponding to one or more constant pressure control periods shown in FIG. 6.

The second pulse wave signal may be a peripheral arterial pulse wave corresponding to one or more constant pressure control periods shown in FIG. 13.

In a possible implementation, the cardiovascular risk includes any one or more of the following: a heart rate, vascular wall elasticity, a cardiac ejection capability, and peripheral vascular resistance.

S2203: The wearable device outputs the first blood pressure measurement value and the cardiovascular risk.

That the wearable device outputs the first blood pressure measurement value and the cardiovascular risk may include: The wearable device displays the first blood pressure measurement value and the cardiovascular risk, or plays the first blood pressure measurement value and the cardiovascular risk through speech, or sends the first blood pressure measurement value and the cardiovascular risk to another device for display.

According to the method, the wearable device can further monitor a cardiovascular disease risk of the user when obtaining a peripheral arterial blood pressure value, to prevent occurrence of a cardiovascular disease.

In a possible implementation, the method further includes: The wearable device obtains a third pulse wave signal based on the second pulse wave signal and a first target model. The wearable device obtains a second blood pressure measurement value based on the first blood pressure measurement value and the third pulse wave signal.

The first blood pressure measurement value may be a central arterial blood pressure value. The central arterial blood pressure value may be aortic blood pressure, and is pressure directly applied to the heart and other organs. The aorta is closer to the heart and the brain. Therefore, compared with the peripheral arterial blood pressure value, the central arterial blood pressure value is of greater significance in assessing a blood pressure risk of the user.

The third pulse wave signal may be a central pulse wave signal. The first target model may be the target model 1 shown in the embodiment of FIG. 17.

According to the method, the wearable device can further obtain the central arterial blood pressure value when obtaining the peripheral arterial blood pressure value.

In a possible implementation, before the wearable device obtains the first blood pressure measurement value based on the first pulse wave signal, the method further includes: Within a third time period, the wearable device inflates the airbag and obtains a fourth pulse wave signal, where pressure in the airbag gradually increases over time within the third time period. The wearable device splices the first pulse wave signal and the fourth pulse wave signal in chronological order to obtain a fifth pulse wave signal, where the fifth pulse wave signal is used to obtain the first blood pressure measurement value.

Optionally, the wearable device may obtain the first blood pressure measurement value based on the fifth pulse wave signal and airbag pressure signals that correspond to the first time period and the third time period.

Measuring blood pressure by the wearable device is a complete process of inflation and deflation. In the complete process of inflation and deflation, the wearable device may obtain pulse wave signals in a plurality of linear pressure increase periods. The wearable device may splice the pulse wave signals in the plurality of linear pressure increase periods into a complete pulse wave signal, and obtain the first blood pressure measurement value based on the complete pulse wave signal.

The third time period may be a time period corresponding to one or more linear pressure increase periods shown in FIG. 6.

The fourth pulse wave signal may be a peripheral arterial pulse wave corresponding to one or more linear pressure increase periods shown in FIG. 13.

The fifth pulse wave signal may be a peripheral arterial pulse wave shown in FIG. 14.

In a possible implementation, before the wearable device outputs the first blood pressure measurement value and the cardiovascular risk, the method further includes: Within a fourth time period, the wearable device inflates the airbag and obtains a sixth pulse wave signal, where within the fourth time period, pressure in the airbag remains unchanged, or pressure in the airbag fluctuates within the first range. That the wearable device obtains the second pulse wave signal specifically includes: The wearable device selects the second pulse wave signal with a good signal from the second pulse wave signal.

Optionally, the wearable device may obtain the third pulse wave signal based on the second pulse wave signal and the first target model, and the wearable device obtains, based on the third pulse wave signal, parameter information for assessing the cardiovascular risk.

Measuring blood pressure by the wearable device is a complete process of inflation and deflation. In the complete process of inflation and deflation, the wearable device may obtain pulse wave signals in a plurality of constant pressure control periods.

In a possible implementation, the wearable device may select a pulse wave signal with a good signal from the pulse wave signals in the plurality of constant pressure control periods, obtain a central pulse wave signal based on the pulse wave signal, and then assess the cardiovascular risk based on the central pulse wave signal.

In another possible implementation, the wearable device may obtain a central pulse wave signal based on a pulse wave signal in each constant pressure control period, then assess the cardiovascular risk based on each central pulse wave signal, and finally average a plurality of cardiovascular risks to obtain a final cardiovascular risk.

The fourth time period may be a time period corresponding to one or more constant pressure control periods shown in FIG. 6.

The sixth pulse wave signal may be a peripheral arterial pulse wave corresponding to one or more constant pressure control periods shown in FIG. 13.

For details, refer to the descriptions in the embodiments of FIG. 16 to FIG. 18.

In a possible implementation, the blood pressure measurement apparatus further includes a drive circuit, a micropump, and a pressure sensor. The drive circuit is configured to control an air inflation rate at which the micropump inflates the airbag. The pressure sensor is configured to obtain an arterial pulse wave signal. An air release rate of the micropump is a first air release rate. That the wearable device inflates the airbag and obtains the first pulse wave signal specifically includes: The wearable device controls the drive circuit to be in a first duty cycle, controls, through the drive circuit, the micropump to inflate the airbag at a first air inflation rate, and obtains the first pulse wave signal through the pressure sensor, where the first air inflation rate is greater than the first air release rate.

With reference to the first aspect, in a possible implementation, that the wearable device inflates the airbag and obtains the second pulse wave signal specifically includes: The wearable device controls the drive circuit to be in a second duty cycle, controls, through the drive circuit, the micropump to inflate the airbag at a second air inflation rate, and obtains the second pulse wave signal through the pressure sensor, where the second duty cycle is less than the first duty cycle, the second air inflation rate is less than the first air inflation rate, and the second air inflation rate is equal to the first air release rate.

With reference to the first aspect, in a possible implementation, the blood pressure measurement apparatus further includes an air valve, and the air valve is configured to exhaust air in the airbag. In a process of obtaining, by the wearable device, the first pulse wave signal and the second pulse wave signal, the method further includes: The wearable device closes the air valve.

In some embodiments, the wearable device may control, by adjusting an inflation speed of the micropump, pressure in the airbag to increase or remain constant, to obtain the first blood pressure measurement value and the cardiovascular risk.

For details, refer to the descriptions in the embodiments of FIG. 7 to FIG. 9B.

In a possible implementation, the blood pressure measurement apparatus further includes a drive circuit, a micropump, a pressure sensor, and an air valve. The drive circuit is configured to control an air inflation rate at which the micropump inflates the airbag. The pressure sensor is configured to obtain an arterial pulse wave signal. The air valve is configured to exhaust air in the airbag. An air release rate of the micropump is a first air release rate. That the wearable device inflates the airbag and obtains the first pulse wave signal specifically includes:

The wearable device controls the drive circuit to be in a first duty cycle, controls, through the drive circuit, the micropump to inflate the airbag at a first air inflation rate, closes the air valve, and obtains the first pulse wave signal through the pressure sensor, where the first air inflation rate is greater than the first air release rate.

With reference to the first aspect, in a possible implementation, that the wearable device inflates the airbag and obtains the second pulse wave signal specifically includes: The wearable device controls the drive circuit to be in the first duty cycle, controls, through the drive circuit, the micropump to inflate the airbag at the first air inflation rate, opens the air valve, and exhausts air in the airbag at a second air release rate through the air valve, where the first air inflation rate is equal to a sum of the first air release rate and the second air release rate.

In some embodiments, the wearable device may adjust an inflation speed of the micropump to remain constant, and control an air release speed of the air valve to enable pressure in the airbag to increase or remain constant, to obtain the first blood pressure measurement value and the cardiovascular risk.

For details, refer to the descriptions in the embodiments of FIG. 10 to FIG. 12B.

The foregoing descriptions are merely some embodiments and implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

It can be understood that the user interfaces described in embodiments of this application are merely example interfaces, and constitute no limitation on the solutions of this application. In other embodiments, the user interfaces may use different interface layouts or may include more or fewer controls, or other function options may be added or omitted. Provided that the user interfaces are based on a same inventive idea provided in this application, all of the user interfaces fall within the protection scope of this application.

It should be noted that, if no contradiction or conflict occurs, any features or any parts of any feature in any embodiment of this application may be combined, and a combined technical solution also falls within the scope of embodiments of this application.

**In** conclusion, the foregoing embodiments are merely intended to describe the technical solutions of this application, but not to limit this application. Although this application is described in detail with reference to the foregoing embodiments, persons of ordinary skill in the art should understand that they may still make modifications to the technical solutions described in the foregoing embodiments or make equivalent replacements to some technical features thereof, without departing from the scope of the technical solutions in embodiments of this application.

## Claims

1. A blood pressure measurement method, wherein the method is applied to a wearable device, the wearable device comprises a blood pressure measurement apparatus, the blood pressure measurement apparatus comprises an airbag, and the method comprises:
within a first time period, inflating, by the wearable device, the airbag and obtaining a first pulse wave signal, wherein pressure in the airbag gradually increases over time within the first time period, and the first pulse wave signal is used to obtain a first blood pressure measurement value;
within a second time period, inflating, by the wearable device, the airbag and obtaining a second pulse wave signal, wherein within the second time period, pressure in the airbag remains unchanged, or pressure in the airbag fluctuates within a first range, and the second pulse wave signal is used to assess a cardiovascular risk; and
outputting, by the wearable device, the first blood pressure measurement value and the cardiovascular risk.

2. The method according to claim 1, wherein the method further comprises:
obtaining, by the wearable device, a third pulse wave signal based on the second pulse wave signal and a first target model; and
obtaining, by the wearable device, a second blood pressure measurement value based on the first blood pressure measurement value and the third pulse wave signal.

3. The method according to claim 1 or 2, wherein the cardiovascular risk comprises any one or more of the following: a heart rate, vascular wall elasticity, a cardiac ejection capability, and peripheral vascular resistance.

4. The method according to any one of claims 1 to 3, wherein before outputting, by the wearable device, the first blood pressure measurement value and the cardiovascular risk, the method further comprises:
within a third time period, inflating, by the wearable device, the airbag and obtaining a fourth pulse wave signal, wherein pressure in the airbag gradually increases over time within the third time period;
splicing, by the wearable device, the first pulse wave signal and the fourth pulse wave signal in chronological order to obtain a fifth pulse wave signal, wherein the fifth pulse wave signal is used to obtain the first blood pressure measurement value; and
obtaining, by the wearable device, the first blood pressure measurement value based on the fifth pulse wave signal and airbag pressure signals that correspond to the first time period and the third time period.

5. The method according to any one of claims 1 to 3, wherein before outputting, by the wearable device, the first blood pressure measurement value and the cardiovascular risk, the method further comprises:
within a fourth time period, inflating, by the wearable device, the airbag and obtaining a sixth pulse wave signal, wherein within the fourth time period, pressure in the airbag remains unchanged, or pressure in the airbag fluctuates within the first range; and
obtaining, by the wearable device, the second pulse wave signal specifically comprises:
selecting, by the wearable device, the second pulse wave signal with a good signal from the second pulse wave signal and the sixth pulse wave signal.

6. The method according to any one of claims 1 to 5, wherein the blood pressure measurement apparatus further comprises a drive circuit, a micropump, and a pressure sensor, the drive circuit is configured to control an air inflation rate at which the micropump inflates the airbag, the pressure sensor is configured to obtain an arterial pulse wave signal, an air release rate of the micropump is a first air release rate, and inflating, by the wearable device, the airbag and obtaining the first pulse wave signal specifically comprises:
controlling, by the wearable device, the drive circuit to be in a first duty cycle, controlling, through the drive circuit, the micropump to inflate the airbag at a first air inflation rate, and obtaining the first pulse wave signal through the pressure sensor, wherein the first air inflation rate is greater than the first air release rate.

7. The method according to claim 6, wherein inflating, by the wearable device, the airbag and obtaining the second pulse wave signal specifically comprises:
controlling, by the wearable device, the drive circuit to be in a second duty cycle, controlling, through the drive circuit, the micropump to inflate the airbag at a second air inflation rate, and obtaining the second pulse wave signal through the pressure sensor, wherein the second duty cycle is less than the first duty cycle, the second air inflation rate is less than the first air inflation rate, and the second air inflation rate is equal to the first air release rate.

8. The method according to claim 6 or 7, wherein the blood pressure measurement apparatus further comprises an air valve, the air valve is configured to exhaust air in the airbag, and in a process of obtaining, by the wearable device, the first pulse wave signal and the second pulse wave signal, the method further comprises:
closing, by the wearable device, the air valve.

9. The method according to any one of claims 1 to 5, wherein the blood pressure measurement apparatus further comprises a drive circuit, a micropump, a pressure sensor, and an air valve, the drive circuit is configured to control an air inflation rate at which the micropump inflates the airbag, the pressure sensor is configured to obtain an arterial pulse wave signal, the air valve is configured to exhaust air in the airbag, an air release rate of the micropump is a first air release rate, and inflating, by the wearable device, the airbag and obtaining the first pulse wave signal specifically comprises:
controlling, by the wearable device, the drive circuit to be in a first duty cycle, controlling, through the drive circuit, the micropump to inflate the airbag at a first air inflation rate, closing the air valve, and obtaining the first pulse wave signal through the pressure sensor, wherein the first air inflation rate is greater than the first air release rate.

10. The method according to claim 9, wherein inflating, by the wearable device, the airbag and obtaining the second pulse wave signal specifically comprises:
controlling, by the wearable device, the drive circuit to be in the first duty cycle, controlling, through the drive circuit, the micropump to inflate the airbag at the first air inflation rate, opening the air valve, and exhausting air in the airbag at a second air release rate through the air valve, wherein the first air inflation rate is equal to a sum of the first air release rate and the second air release rate.

11. The method according to any one of claims 8 to 10, wherein after obtaining, by the wearable device, the first pulse wave signal and the second pulse wave signal, the method further comprises:
opening, by the wearable device, the air valve and exhausting air in the airbag through the air valve.

12. A wearable device, wherein the wearable device comprises a blood pressure measurement apparatus, a memory, and a processor, the blood pressure measurement apparatus and the memory are coupled to the processor, the memory is configured to store a computer program, and when the processor invokes the computer program, the wearable device is enabled to perform the method according to any one of claims 1 to 11.

13. A computer-readable storage medium, comprising instructions, wherein when the instructions are run on a wearable device, the wearable device is enabled to perform the method according to any one of claims 1 to 11.
